# FASCICULE DE BREVET EUROPEEN

(11) **EP 0 612 356 B1**
(45) Date de publication et mention de la délivrance du brevet: **10.04.1996**
(21) Numéro de dépôt: 93900219.2
(22) Date de dépôt: 30.10.1992
(51) Int. Cl.: C12Q 1/68

(54) **SEQUENCES NUCLEOTIDIQUES HYBRIDANT SPECIFIQUEMENT AVEC LES SOUCHES BACTERIENNES DU COMPLEXE MYCOBACTERIUM AVIUM-INTRACELLULARE**
NUKLEOTID-SEQUENZEN DIE SPEZIFISCH MIT BAKTERIEN DES MYKOBAKTERIUM AVIUM-INTERCELLULARE - KOMPLEXES HYBRIDIEREN
NUCLEOTIDE SEQUENCES HYBRIDIZING SPECIFICALLY WITH BACTERIAL STRAINS OF THE COMPLEX $i(MYCOBACTERIUM AVIUM-INTRACELLULARE)

(30) Priorité: 31.10.1991 FR 9113504
(43) Date de publication de la demande: 31.08.1994
(73) Titulaire: INSTITUT PASTEUR, F-75724 Paris Cédex 15 (FR)
(72) Inventeur: GUESDON, Jean-Luc, F-92310 Sèvres (FR); THIERRY, Dominique, F-92100 Boulogne (FR); VINCENT, Véronique, F-75005 Paris (FR)
(74) Mandataire: Le Guen, Gérard
(86) Numéro de dépôt international: FR9201015
(87) Numéro de publication internationale: WO9309251

(56) Documents cités:
- EP-A- 0 288 306
- WO-A-90/12875
- FR-A- 2 651 505
- MOLECULAR MICROBIOLOGY, vol. 3, no. 7, 1989, Oxford (GB); A.J. HANCE et al., pp. 843-849
- JOURNAL OF CLINICAL MICROBIOLOGY, vol. 25, no. 8, août 1987, Washington, DC (US); T.E. KIEHN et al., pp. 1551-1552

## Description

La présente invention se rapporte a une séquence nucléique spécifique du complexe Mycobacterium avium-intracellulare, ainsi que des fragments particuliers de cette séquence, aptes à jouer le rôle de sondes nucléiques pour la détection d'un acide nucléique spécifique de souches appartenant au complexe Mycobacterium avium-intracellulare ou d'amorces nucléiques dans l'amplification d'ADN ou d'ARN provenant de souches du complexe Mycobacterium avium-intracellulare dans un échantillon biologique.

L'invention concerne également une méthode de détection de souches appartenant au complexe Mycobacterium avium-intracellulare dans un échantillon biologique utilisant les fragments d'acides nucléiques, ainsi qu'un nécessaire pour la mise en oeuvre de cette méthode.

Le nombre des infections provoquées par des mycobactéries opportunistes augmente en partie à cause de leur fréquence chez les malades atteints du Sida. D'après les études bactériologiques réalisées ces dernières années, les mycobactéries opportunistes les plus importantes sont : *M*. *kansasii, M*. *xenopi, M*. *simiae* et surtout les mycobactéries appartenant au complexe : M. avium-intracellulare (complexe MAI). Il existe cependant des variations géographiques dans l'incidence de l'infection par ces diverses mycobactéries opportunistes.

Dans une étude récente réalisée par le Laboratoire de bactériologie et virologie du groupe hospitalier Pitié-Salpêtrière dirigé par le Professeur J. Grosset, il a été montré que parmi les 709 malades atteints de Sida dont le sang a été mis en culture (2460 échantillons) 63 (8,9 %) ont eu des hémocultures positives à mycobactéries. D'autre part, 84,1 % des hémocultures positives contenaient M. avium-intra cellulare et 11,1 % M. tuberculosis.

M. avium et M. intracellulare sont deux espèces phénotypiquement proches indiscernables par identification classique, et sont donc regroupées en complexe MAI. Par complexe MAI au sens de la présente invention, on entend les souches telles que définies par SAITO et Coll. (J. Clin. Microbiol., août 1990, 28, p. 1694 - 1697). Ce complexe rassemble des pathogènes pour l'homme et l'animal. Chez l'homme non infecté par les virus HIV, les infections sont surtout à localisation pulmonaire chez l'adulte et ganglionaire chez l'enfant. Des infections à MAI sont diagnostiquées chez 50 à 90 % des malades atteints de SIDA. Les localisations sont alors préférentiellement digestives et disséminées. MAI est aussi un pathogène d'importance en médecine vétérinaire touchant les volailles, bovins, caprins, ovins,... Les pathogènes pour l'animal, M. paratuberculosis et M. silvaticum ont récemment été introduits dans la nomenclature comme sous-espèces de M. avium (Thorel et al, Int. J. Syst. Bactériol., 1990, 40, 254-260). Il faut également noter que des souches de M. paratuberculosis ont été isolées chez des malades atteints de la maladie de Crohn.

Par la sérologie on reconnaît 28 sérotypes à l'intérieur de MAI. Actuellement sont classés comme M. avium les sérotypes 1 à 6, 8 à 11 et 21 et M. intracellulare les sérotypes 7, 12 à 20 et 25. Il persiste une incertitude quant à la position taxonomique des sérotypes 22 à 24 et 26 à 28. Chez l'homme non infecté par les virus VIH, la distribution des sérotypes est très dispersée alors que chez les malades atteints de SIDA, seuls les sérotypes 1, 4, 8 et 9 sont majoritaires (données du Centre National de Référence pour les Mycobactéries). Cette distribution se rapproche de celle observée chez les animaux où l'on retrouve préférentiellement, les sérotypes 1, 2, 3, 4, 8. La situation épidémiologique française des souches de M. avium isolées de malades atteints du SIDA diffère de celle observée aux Etats Unis ou en Suède. Alors qu'en France, le sérotype 8 est majoritaire, ce sont les sérotypes 4 ou 6 que l'on retrouve dans ces deux autres pays.

Les sérotypes (ou sérovars) mentionnés dans le présent brevet sont tels que définis par P.J. Brennan et coll. (Identification of A typical Mycobacteria by Thin-Layer Chromatography of Their Surface Antigens, Journal of Clinical Microbiology, Oct. 1978, p. 374-379.

Les mycobactéries opportunistes posent de multiples défis au microbiologiste, à l'immunologiste et au clinicien. En effet, contrairement à M. tuberculosis qui présente une population bactérienne homogène, les souches du complexe M. avium-intracellulare sont composées de cellules donnant divers types de colonies à la culture ; une discordance entre la réponse de malades à la chimiothérapie antituberculeuse et les résultats de l'antibiogramme in vitro a été observée, remettant en cause le rôle des antibiogrammes (en tout état de cause les infections à MAI répondent peu ou mal aux divers traitements antibiotiques, antituberculeux ou autres) ; les mycobactéries sont à l'origine de la sensibilisation non spécifique à la tuberculine et peuvent également affecter la réponse immunitaire.

En laboratoire, la difficulté consiste à isoler les mycobactéries opportunistes à partir de divers prélèvements puis à déterminer l'espèce. La présence ubiquitaire des mycobactéries opportunistes dans l'environnement rend la distinction entre colonisation et infection souvent difficile. C'est la raison pour laquelle il est important de se référer à des critères cliniques et bactériologiques précis avant de décider de la signification clinique d'une mycobactérie isolée d'un prélèvement. Par conséquent, il est indispensable d'établir une coopération de bonne qualité entre les cliniciens et les bactériologistes et de posséder des techniques fiables et spécifiques pour identifier les mycobactéries opportunistes. Il est évident que les espèces mycobactériennes isolées et identifiées dans les produits pathologiques sont liées aux techniques d'isolement et aux schémas utilisés aujourd'hui pour leur identification. Les bactériologistes savent que l'identification des mycobactéries opportunistes est rendue difficile par plusieurs éléments : certaines espèces sont plus ou moins sensibles aux techniques de décontamination nécessaire avant la culture, plusieurs espèces ne s'isolent en primoculture que dans certaines conditions, enfin, certaines techniques d'identification basées sur des propriétés phénotypiques ne sont pas suffisamment fiables et peuvent conduire à une identification erronée. D'autre part le statut taxonomique de certaines mycobactéries apparentées au complexe MAI reste encore incertain et dans ce cas l'identification ne peut être établie.

Les mycobactéries du complexe M. avium-intracelllulare peuvent comme M. tuberculosis être responsables d'adénites chez l'enfant. Il est fréquent que le clinicien donne un traitement initial antituberculeux dans l'attente du diagnostic microbiologique qui peut nécessiter plusieurs semaines, ce traitement étant arrêté dès lors que le diagnostic microbiologique est acquis.

Afin de pallier ces erreurs d'interprétation et de permettre un diagnostic rapide, les inventeurs à l'origine de la présente invention se sont donné pour objectif d'isoler des séquences d'ADN spécifiques des mycobactéries appartenant au complexe M. avium-intracellulare et de développer à partir de ces séquences une méthode d'identification basée sur la technique PCR et la technique d'hybridation moléculaire. Ils ont donc recherché des séquences utilisables aussi bien pour le diagnostic que pour l'épidémiologie moléculaire.

Récemment des approches utilisant l'hybridation moléculaire pour identifier les espèces M. avium-intracellulare ont été proposées, cependant la plupart de ces méthodes ne permettent l'identification qu'après culture car elles ne sont pas suffisamment sensibles pour détecter ces mycobactéries directement dans les prélèvements biologiques. C'est le cas par exemple de la sonde d'ADNc complémentaire de séquences d'ARN ribosomique et commercialisée par Gene-Probe (San Diego, Californie), voir WO 84/02721, WO 88/03957, Musial, C.E. et al. (J. Clin. Microbiol., 1988, 26, 2120-2123). De plus, des souches mycobactériennes selon les critères de Saito et al. (J. Clin. Microbiol, 1990, 28, 1694-1697) ne sont pas détectables en utilisant les sondes commercialisées par Gene-Probe. D'autres auteurs ont trouvé des résultats similaires : Enns, R.K. (Lab. Med. 1988, 19, 295-300), Sherman, I. et al. (J. Clin. Microbiol., 1989, 27, 241-244).

FR-2 645 878 décrit en outre une séquence nucléique de 383 paires de bases homologues chez au moins 5 espèces de mycobactéries dont M. tuberculosis.

Cette séquence présente toutefois l'inconvénient de ne pas être spécifique du complexe M. avium-intracellulare.

Une méthode plus sensible et plus spécifique était donc nécessaire pour établir un diagnostic fiable des mycobactérioses atypiques et une identification précise des diverses mycobactéries opportunistes.

La présente invention a pour but de fournir une telle méthode ainsi que les éléments mettant en oeuvre cette méthode.

La présente invention a ainsi pour objet une séquence d'acide nucléique qui hybride spécifiquement avec une séquence d'acide nucléique génomique des souches appartenant au complexe Mycobacterium avium-intracellulare choisie parmi la séquence nucléotidique SEQ ID N°1, la séquence nucléotidique SEQ ID N°2, des séquences complémentaires de celles-ci, ainsi que les séquences qui en diffèrent par mutation, insertion, délétion ou substitution d'une ou plusieurs bases, et qui n'hybride pas avec les acides nucléiques des mycobactéries n'appartenant pas audit complexe.

La présente invention a également pour objet une séquence d'acide nucléique choisie parmi les séquences nucléotidiques SEQ ID N°1 et SEQ ID N°2, leurs séquences complémentaires ainsi que les séquences qui en diffèrent par mutation, insertion, délétion ou substitution d'une ou plusieurs bases et qui ont la même specificité.

Les séquences d'acide nucléique définies ci-dessus peuvent être des séquences d'ADN ou des séquences d'ARN.

La taille exacte du fragment de séquence SEQ ID N°1 (fragment I6) est de 1642 paires de bases. La recherche dans les banques de données EMBL et Genbank n'a fait ressortir aucune homologie significative avec les séquences d'ADN déjà connues. Le fragment I6 a été cloné dans le plasmide pUC18 et le plasmide recombiné est nommé pMA01.

Le plasmide pMA01 a été déposé à la Collection nationale de Cultures de Microorganismes le 28 août 1991 sous le n° I-1137.

Le fragment de séquence SEQ ID N°2 (fragment I1) utilisé comme sonde sur les différents sérotypes M. avium-intracellulare montre une hybridation sur les sérotypes 2, 3, 7, 12, 13, 14, 15, 16, 17, 18, 19, 20, 23, 24 et 25. Le fragment Il hybride avec des souches M.intra-cellulare et les souches M. avium sérotypes 2 et 3.

Aujourd'hui, on considère comme M. avium, les sérovars 1, 2, 3, 4, 5, 6, 8, 9, 10, 11 et 21 et comme M. intracellulare les sérovars 7, 12, 13, 14, 15, 16, 17, 18, 19, 20 et 25.

Le fragment I1 dont la taille est égale à 2004 paires de bases, a été cloné dans le plasmide pUC18 ; le plasmide recombiné est nommé pMA02.

Le plasmide pMA02 a été déposé à la Collection Nationale de Cultures de Microorganismes le 28 août 1991 sous le n° I-1138.

Ces plasmides ainsi que de façon plus générale un vecteur de clonage contenant une séquence d'acide nucléique telle que définie précédemment consituent un autre objet de l'invention.

Une interrogation de la banque de données ne révèle aucune homologie de la séquence de Il avec des séquences connues.

Les fragments I6 et I1, des parties ou des variants fonctionnellement équivalents de ceux-ci peuvent être utilisés dans des techniques d'hybridation moléculaire pour la détection et l'identification des espèces du complexe M. avium-intracellulare. Les variants fonctionnellement équivalents comprennent des séquences dans lesquelles des paires de bases ont été mutées, délétées, insérées, ou substituées sans que les propriétés essentielles à la spécificité de ces fragments soient affectées.

Un fragment d'acide nucléique de 700 paires de bases obtenus en coupant le fragment initial 16 par les enzymes de restriction SalI et EcoRI a été utilisé comme sonde sur l'ADN des différents sérotypes de M. avium. Une hybridation n'est observée qu'avec l'ADN des souches M. avium portant les sérotypes 1, 2, 3, 4, 5, 6, 8, 9, 10, 11 et 21. Il est important de noter que cette sonde de 700 paires de bases permet de détecter les sérotypes de M. avium les plus souvents rencontrés chez les malades atteints de SIDA.

Les séquences I6 et I1 ou les fragments obtenus à partir de ces séquences peuvent également être utilisés pour sélectionner des amorces pour la technique PCR.

Cette technique nécessite le choix de paires d'oligonucléotides encadrant le fragment qui doit être amplifié (brevet U.S. n° 4 683 202). Ces amorces oligodésoxyribonucléotidiques ou oligoribonucléotidiques ont avantageusement une longueur comprise entre 18 et 30 et de préférence 18 et 22 nucléotides. L'une des deux amorces est complémentaire du brin (+) de la matrice et l'autre amorce est complémentaire du brin (-). Il est important que ces amorces ne possèdent pas de structure secondaire ou de séquence complémentaire l'une de l'autre. D'autre part la longueur et la séquence de chaque amorce doivent être choisies de manière à ce que les amorces ne s'hybrident avec d'autres acides nucléiques provenant de cellules procaryotes ou eucaryotes, en particulier avec les acides nucléiques des mycobactéries n'appartenant pas au complexe M. avium-intracellulare et avec l'ADN ou l'ARN humain pouvant éventuellement contaminer le prélèvement.

Les amplimères selectionnés comme amorces spécifiques pour l'amplification de séquences nucléiques de souches appartenant au complexe MAI ont été choisis en suivant la méthode décrite par Griffais et Coll. (Nucleic Acids Res. 1991, 19, 3887-3891).

A partir de la séquence I6, les inventeurs ont choisi des oligonucléotides pour réaliser un test PCR. Grâce à ces oligonucléotides, ils ont obtenu une amplification spécifique de M. avium (sérotypes 1, 2, 3, 4, 5, 6, 8, 9, 10, 11 et 21) et de M. paratuberculosis, aucune amplification n'était visible avec un acide nucléique de M. scrofulaceum, ou de mycobactéries appartenant au complexe tuberculosis.

De même à partir de la séquence I1, les inventeurs ont sélectionné des amorces oligonucléotidiques qui ont permis de mettre au point un test PCR spécifique. Avec les amorces sélectionnées à partir du fragment I1 ils ont observé un fragment amplifié uniquement lorsque l'acide nucléique soumis à l'amplification provenait des souches M. avium-intracellulare portant les sérotypes 2, 3, 7, 12, 13, 14, 15, 16, 17, 18, 19, 20, 23, 24 et 25.

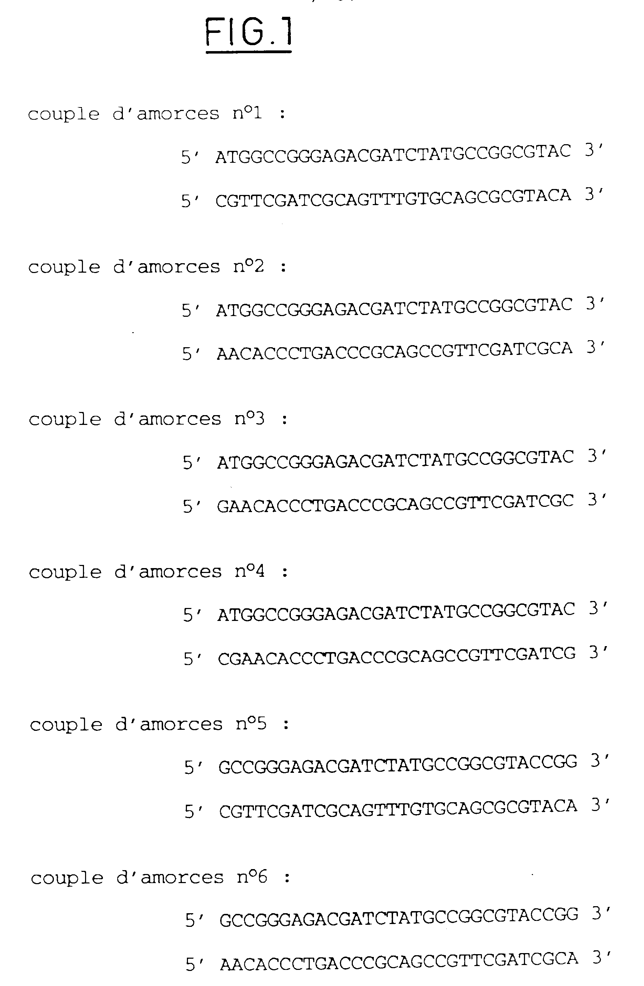

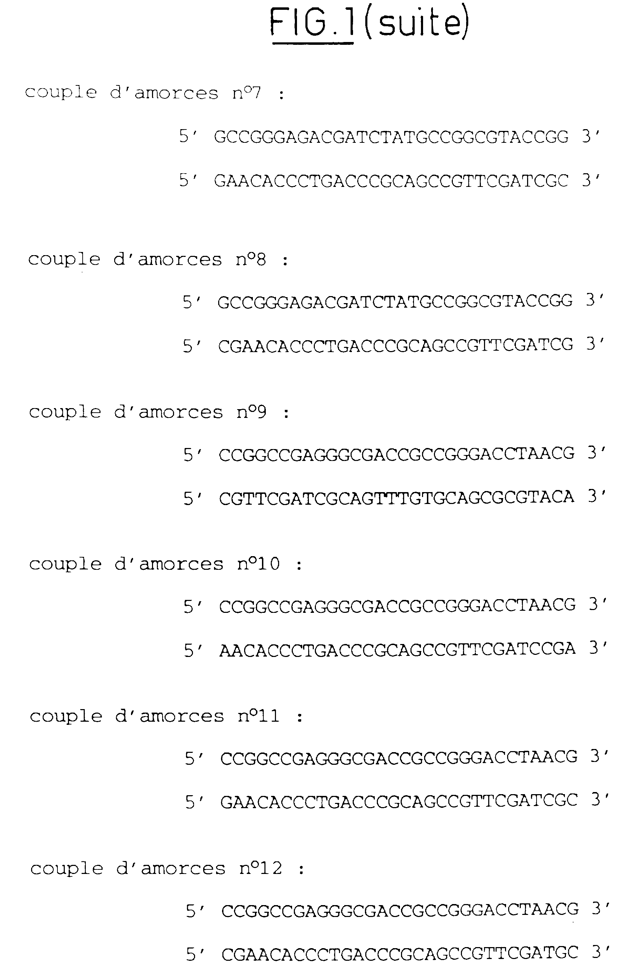

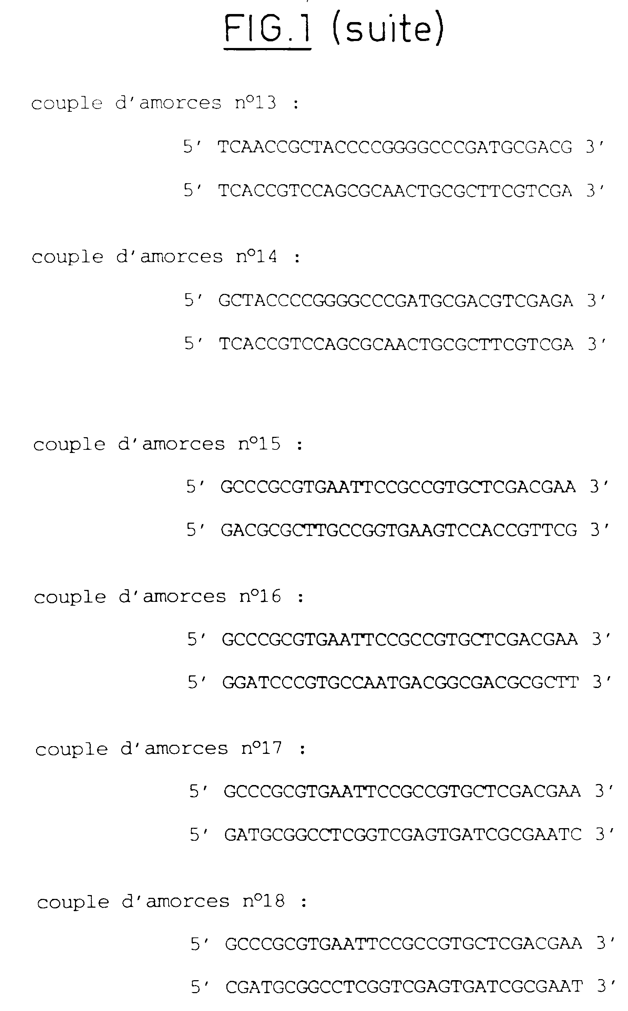

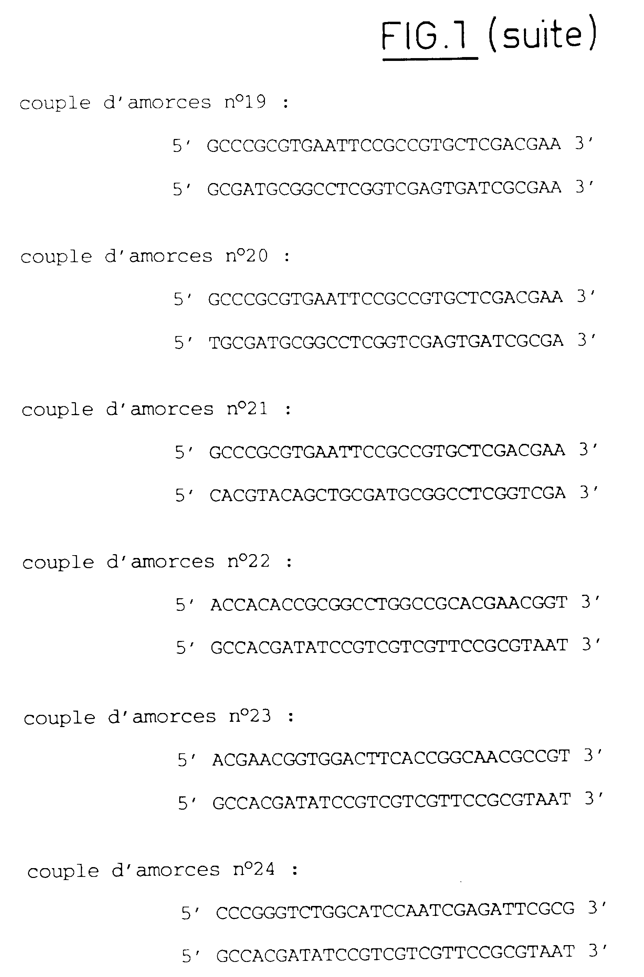

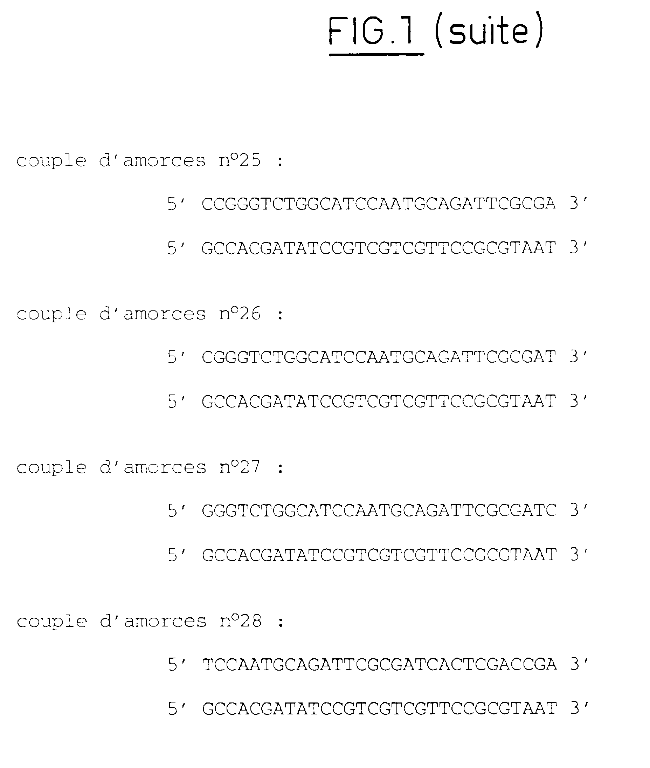

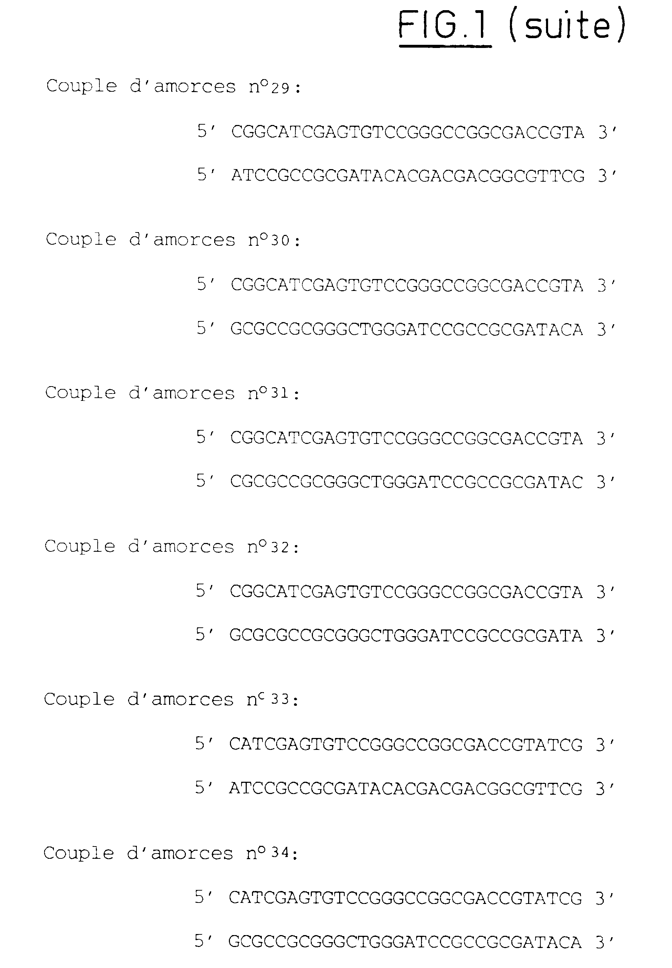

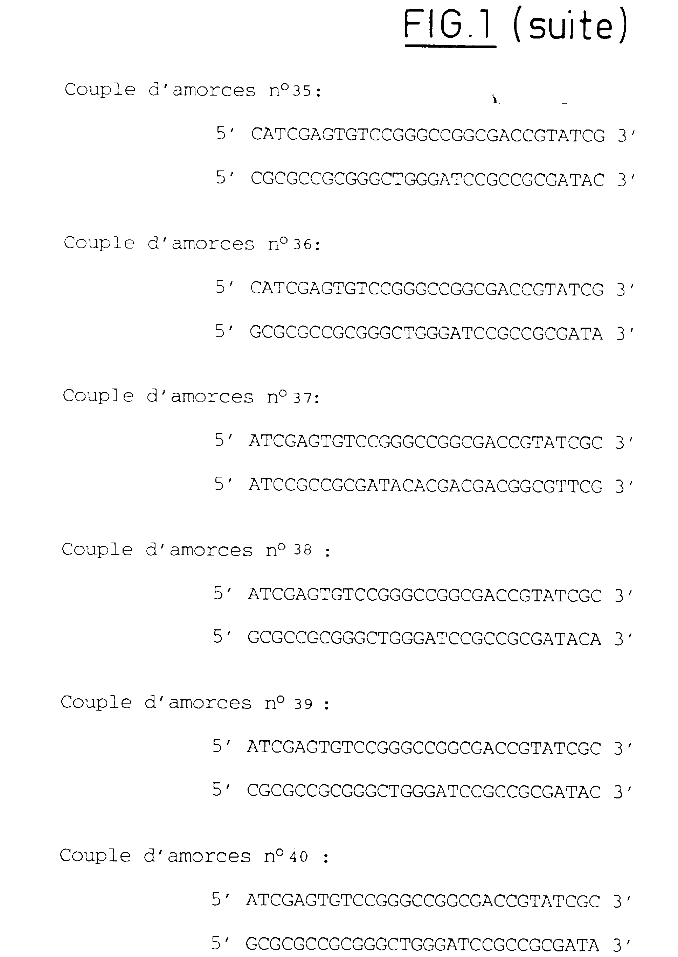

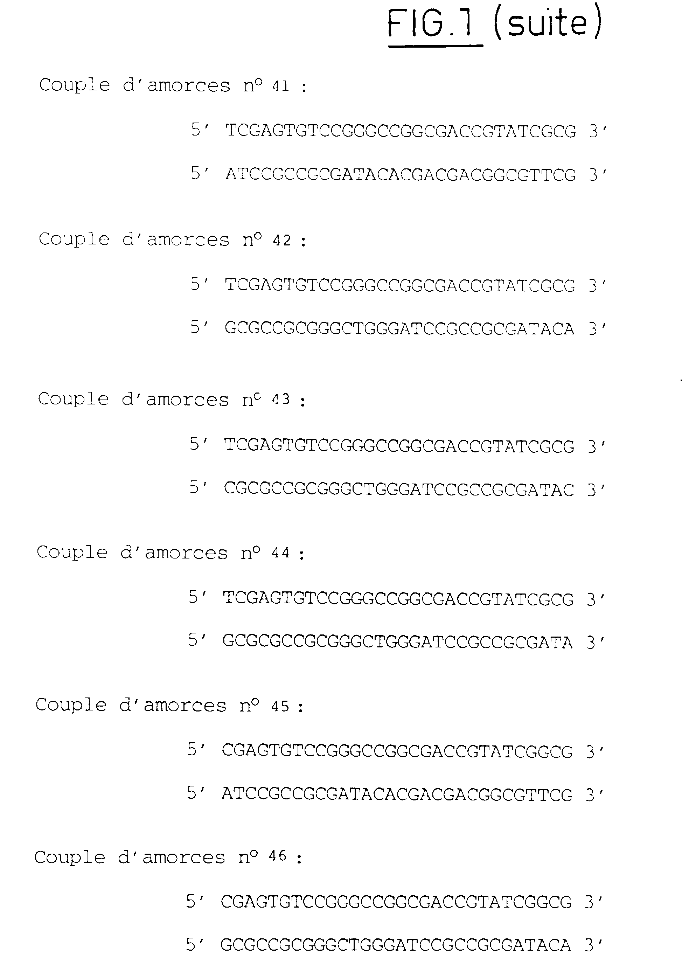

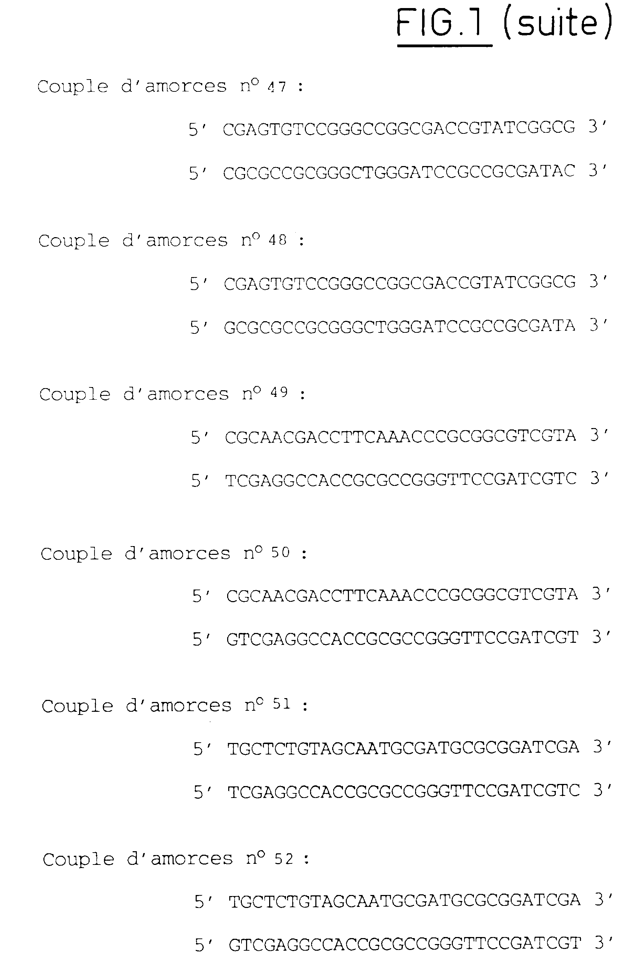

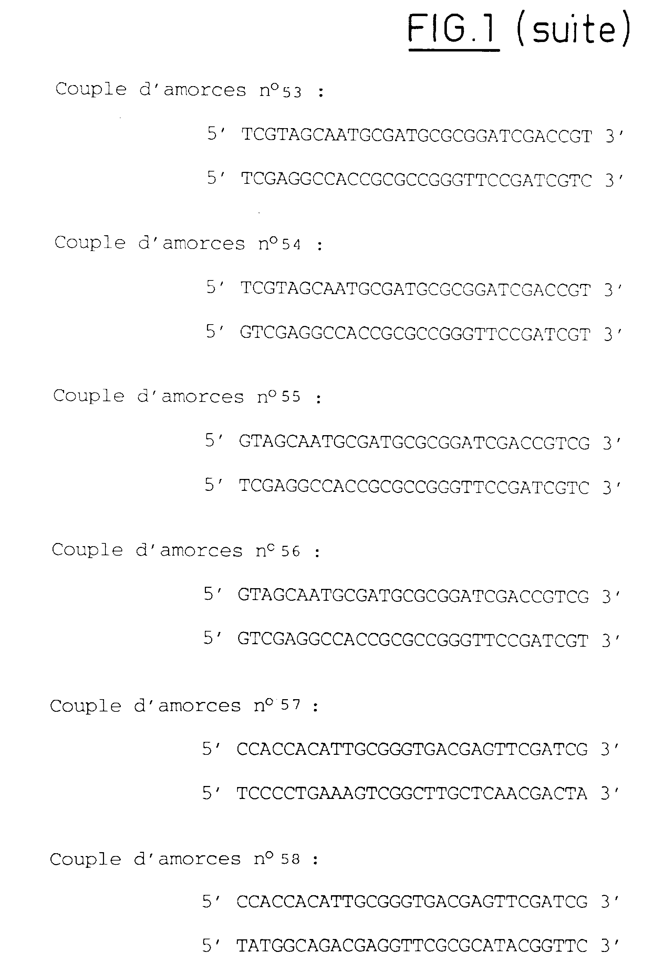

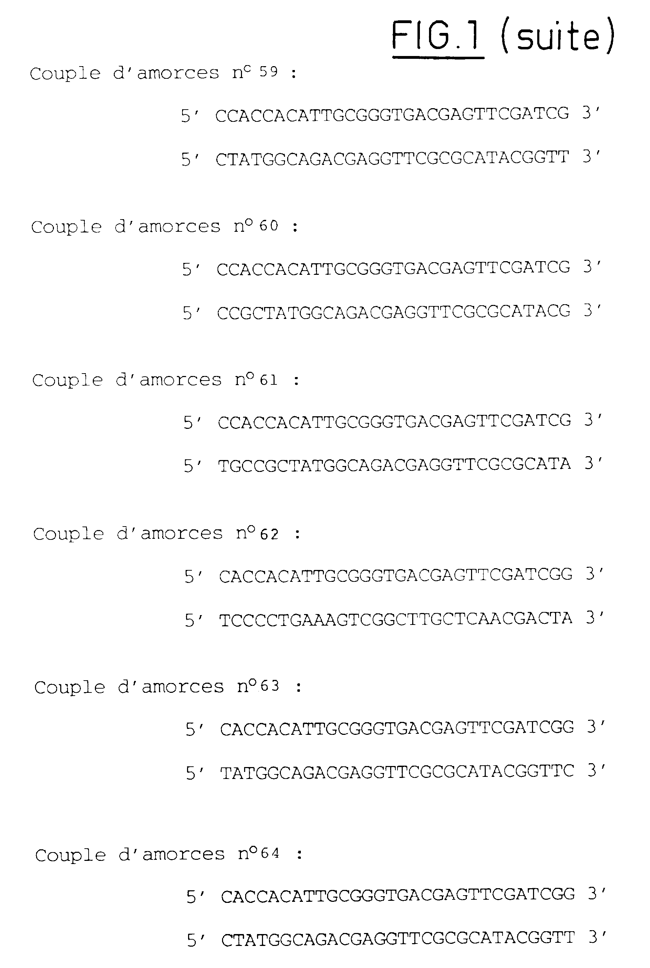

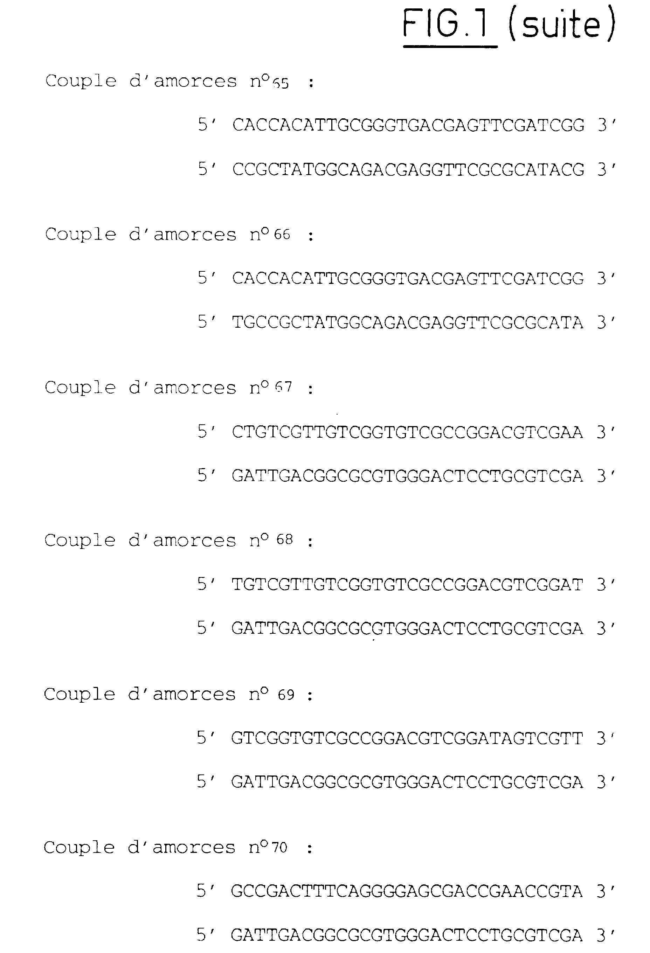

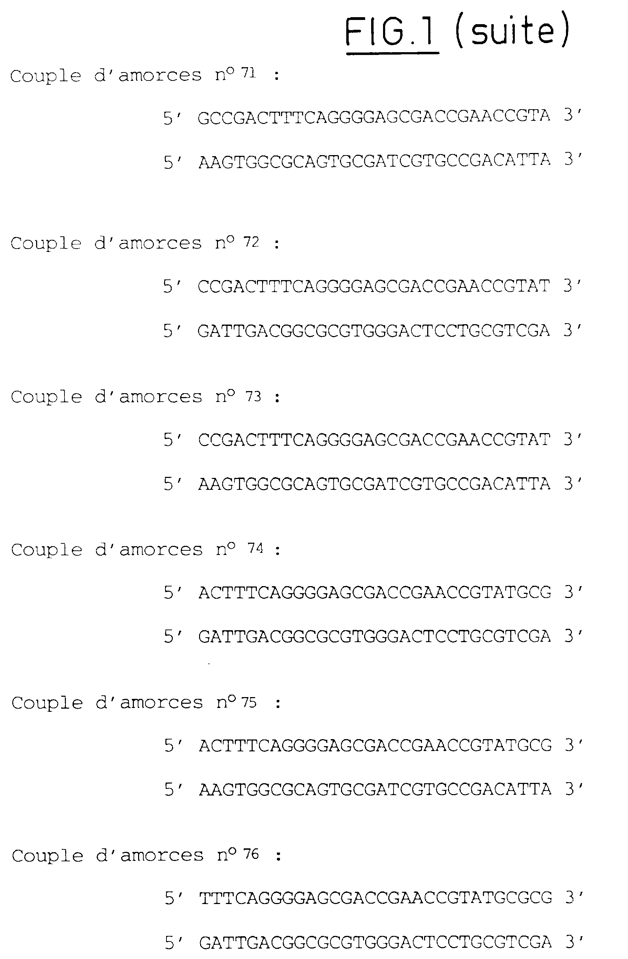

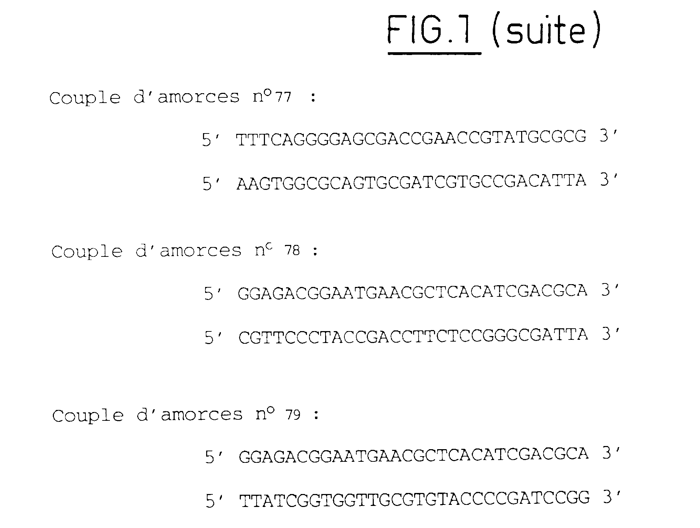

Des couples d'amorces préférés sont représentés à la figure 1. Les paires d'oligonucléotides utilisables comme amorces comprennent de préférence au moins 18 nucléotides consécutifs choisis en conservant l'extrémité 3' des séquences.

Des couples d'amorces tout particulièrement préférés sont représentés par les couples constitués par les oligonucleotides MA1 et MA2 issus de la séquence I6 d'une part et MA3 et MA4 issus de la séquence I1 d'autre part, de séquences :

MA1 : 5'GGC CCG ATG CGA CGT CGA GA3'
MA2 : 5'GCG CAA CTG CGC TTC GTC GA3'
MA3 : 5'GAA CGC CCG TTG CTG GCC ATT CAC GAG GAG3'
MA4 : 5'GCG CAA CAC GGT CGG ACA GGC CTT CCT CGA3'

Les fragments amplifiés peuvent être identifiés après une électrophorèse en gel d'agarose ou de polyacrylamide ou après une électrophorèse capillaire ou encore après une technique chromatographique (filtration sur gel, chromatographie hydrophobe ou sur échangeurs d'ions). La spécificité de l'amplification peut être contrôlée par hybridation moléculaire en utilisant comme sondes, les séquences nucléiques I6 ou I1, des fragments de celles-ci, des plasmides contenant ces séquences ou des fragments de celles-ci, des oligonucléotides complémentaires de ces séquences ou fragments de séquences ou des produits d'amplification. Ces sondes peuvent être marquées ou non par des éléments radioactifs ou par des molécules non radioactives.

Ces sondes comprennent avantageusement au moins 20 nucléotides consécutifs parmi les séquences ou les fragments de séquences mentionnés ci-dessus et constituent un autre objet de l'invention.

Les sondes sont des sondes d'ADN ou des sondes d'ARN.

Les séquences d'acide nucléique décrites dans cette invention peuvent ainsi être utilisées comme sondes pour détecter spécifiquement et de manière directe des souches appartenant au complexe M. avium-intracellulare dans un échantillon biologique. Les séquences non marquées peuvent être utilisées directement comme sondes, cependant les séquences d'acide nucléique sont généralement marqués par un élément radioactif (³p, ³⁵S, ³H,¹⁵I) ou par une molécule non-radioactive (biotine, acétylaminofluorène, digoxigénine, 5-bromo-désoxyuridine) pour obtenir des sondes utilisables pour de nombreuses applications.

Dans ce dernier cas, on pourra utiliser l'une des méthodes de marquage décrits dans FR 2 422 956 et FR 2 518 755. La technique d'hybridation peut être réalisée de diverses manières (Matthews, J.A. et Kricka, L.J., Anal. Biochem. 1988, 169, 1-25). La méthode la plus générale consiste à immobiliser l'acide nucléique extrait des cellules mycobactériennes sur un support (nitrocellulose, nylon, polystyrène...) et à incuber, dans des conditions bien définies, l'acide nucléqiue cible immobilisé avec l'acide nucléique sonde. Après l'hybridation l'excès de sonde est éliminé et les molécules hybrides formées sont détectées par la méthode appropriée (mesure de la radioactivité, de la fluorescence ou de l'activité enzymatique liée à la sonde...).

Dans une autre application, les sondes d'acide nucléique décrites ici peuvent être utilisées comme sondes de capture. Dans ce procédé, la sonde est immobilisée sur un support et sert à capturer par hybridation spécifique l'acide nucléique cible extrait des mycobactéries. Si nécessaire, le support solide est séparé de l'échantillon et le duplex formé entre la sonde de capture et l'acide nucléique cible est ensuite détecté grâce à une seconde sonde de détection marquée par un élément facilement détectable.

Lorsqu'une quantité suffisante d'acide nucléique mycobactérien peut être extraite à partir des prélèvements à analyser, les séquences décrites dans le brevet sont utilisables pour détecter et identifier les souches appartenant au complexe M. avium-intracellulare directement dans ces prélèvements. Dans le cas contraire, une culture rapide en milieu liquide peut être réalisée avant l'extraction de l'acide nucléique mycobactérien, ou bien, la petite quantité d'acide nucléique mycobactérien extrait du prélèvement peut être soumise à la technique PCR.

La présente invention a également pour objet une méthode de détection de la présence des souches appartenant au complexe Mycobacterium avium-intracellulare dans un échantillon biologique, caractérisée par les étapes suivantes :
i) mise en contact de l'échantillon biologique avec un couple de fragments d'acide nucléique, dits amorces, tels que définis précédemment, l'acide nucléique contenu dans l'échantillon ayant été, le cas échéant, préalablement rendu accessible à l'hybridation et dans des conditions permettant une hybridation des amorces à l'acide nucléique des souches appartenant au complexe Mycobacterium avium-intracellulare ;
ii) amplification de l'acide nucléique des souches appartenant au complexe Mycobacterium avium-intra-cellulare ;
iii) mise en évidence de l'amplification de fragments d'acide nucléique correspondant au fragment encadré par les amorces ;
iv) vérification éventuelle de la séquence du fragment amplifié, par exemple par hybridation de sonde spécifique, par séquençage ou par analyse de site de restriction.

La présente invention a, en outre, pour objet un nécessaire pour la détection de la présence de souches appartenant au complexe Mycobacterium avium-intracellulare dans un échantillon biologique, caractérisé en ce qu'il comporte les éléments suivants:
- un couple de fragments d'acide nucléique tels que définis précédemment ;
- les réactifs nécessaires pour effectuer une amplification d'acide nucléique de souches appartenant au complexe Mycobacterium avium-intracellulare ;
- éventuellement un composant permettant de vérifier la séquence du fragment amplifié, plus particulièrement une sonde nucléique telle que définie précédemment.

Ce nécessaire contient plus avantageusement la ou les sondes marquées ou non. Celles-ci peuvent être en solution ou immobilisées sur un support. Le nécessaire peut également contenir les réactifs nécessaires pour la lyse des mycobactéries et l'extraction des acides nucléiques cibles, ainsi que les solutions d'hybridation et de lavage correspondant à la méthode choisie.

L'invention est illustrée plus en détail dans la description qui suit pour laquelle on se référa aux figures annexées sur lesquelles :
- la figure 1 représente les séquences nucléotidiques convenant pour le choix des amorces oligonucléotidiques.
- la figure 2 représente l'analyse en Southern blot de l'ADN cosmidique I6 d'ADN cosmidique 15 µl d'ADN correspondant approximativement à 3 µg ont été digérés avec 10 unités d'enzyme SalI. Après électrophorèse l'ADN a été transféré sur nitrocellulose et hybridé avec les sondes radioactives :
   M. avium (A), M. intracellulare (B), M. tuberculosis (C). La flèche indique le fragment de 1642 paires de bases interressant.
- la figure 3 représente l'analyse en Southern blot de l'ADN cosmidique I1.
   15 µl d'ADN cosmidique correspondant approximativement à 3 µg ont été digérés avec 10 unités d'enzyme SalI. Après électrophorèse l'ADN a été transféré sur nitrocellulose et hybridé avec les sondes radioactives : M. avium (A), M. intracellulare (B), M. tuberculosis (C). La flèche indique le fragment de 2004 paires de bases interressant.

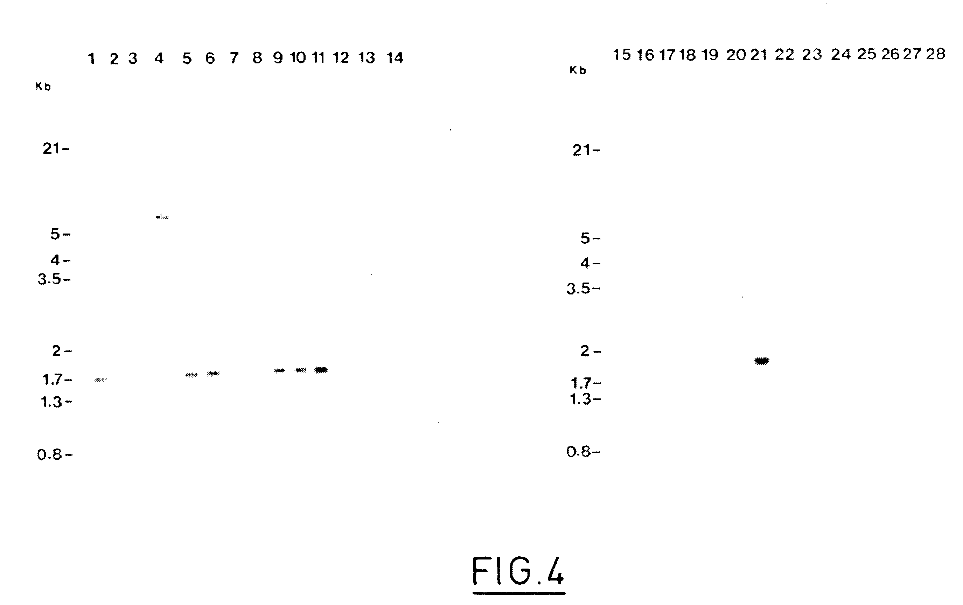

- la figure 4 représente l'analyse en Southern blot de l'ADN de mycobactéries du complexe M. avium - intracellulare.
   2 µg d'ADN ont été digérés avec 10 unités d'enzyme PstI. Après électrophorèse l'ADN a été transféré sur nitrocellulose et hybridé avec 25ng du fragment SalI-ECORI radiomarqué issu de I6. Cette sonde détecte des fragments sur les sérotypes 1, 2, 3, 4, 5, 6, 8, 9, 10, 11 et 21.

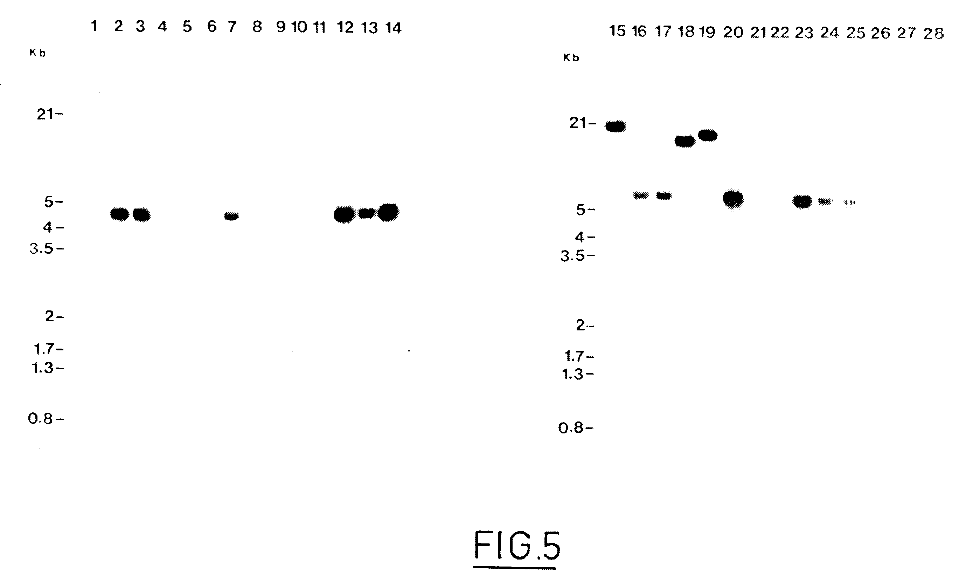

- la figure 5 représente l'analyse en Southern blot de l'ADN de mycobactéries du complexe M. avium - intracellulare.
   2 µg d'ADN ont digérés avec 10 unités d'enzyme PstI. Après électrophorèse l'ADN a été transféré sur nitrocellulose et hybridé avec 25ng du fragment SalI-BamHI radiomarqué issu de I1. Cette sonde détecte des fragments sur les sérotypes 2, 3, 7, 12, 13, 14, 15, 16, 17, 18, 19, 20, 23, 24 et 25.

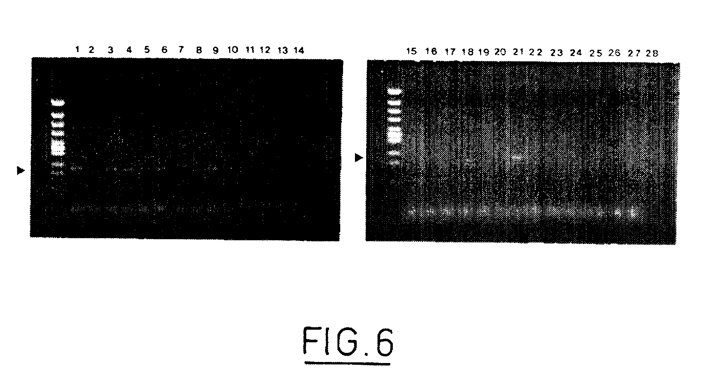

- la figure 6 représente l'analyse sur gel d'agarose des échantillons amplifiés.
   10 µl des échantillons amplifiés avec le couple d'amorces n° 1 décrit à la figure 1 et issues du fragment I6 sont déposés sur un gel d'agarose à 2 % en TAE. Les fragments amplifiés correspondant aux sérotypes 1, 2, 3, 4, 5, 6, 8, 9, 10, 11 et 21 sont visualisés sous UV en présence de bromure d'éthidium.

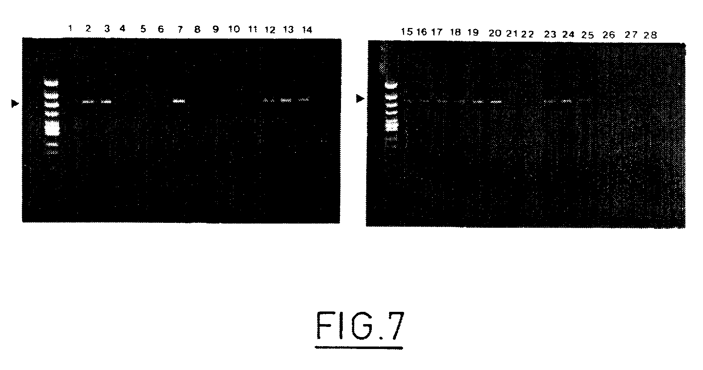

- la figure 7 représente l'analyse sur gel d'agarose des échantillons amplifiés.
   10 µl des échantillons amplifiés avec les oligonucléotides MA3 et MA4 issus du fragment I1 sont déposés sur un gel d'agarose à 2 % en TAE. Les fragments amplifiés correspondant aux sérotypes 2, 3, 7, 12, 13, 14, 15, 16, 17, 18, 19, 20, 23, 24 et 25 sont visualisés sous UV en présence de bromure d'éthidium.

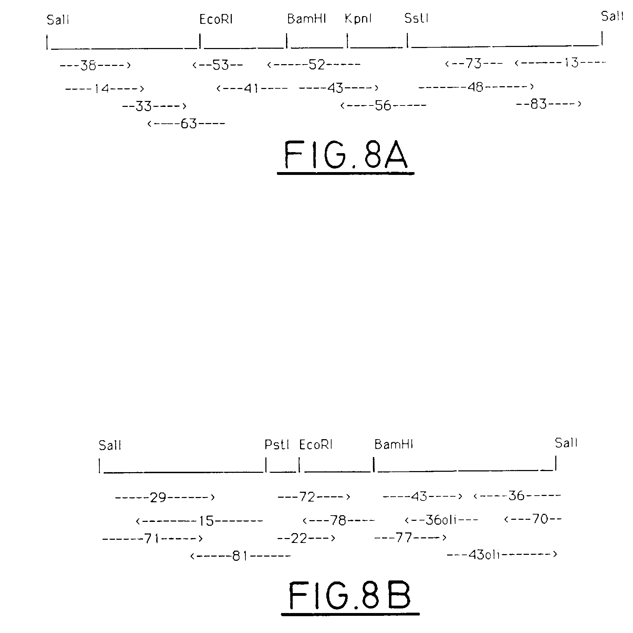

- la figure 8 représente les schémas des stratégies suivies pour séquençage des fragments I6 (A) et I1 (B).

### Construction de la banque génomique M. avium. Criblage de la banque et détermination de la séquence des fragments spécifiques.

L'ADN génomique de M. avium (sérotype 2) est digéré partiellement par l'endonucléase de restriction SalI en faisant agir 0,03U d'enzyme par µg d'ADN dans le tampon recommandé par le fournisseur pendant 1 heure à 37°C. L'ADN génomique ainsi digéré est séparé par électrophorèse sur gel d'agarose à 0,4%. Les fragments dont la longueur est comprise entre 30 et 40 kb sont electroélués et précipités par l'éthanol après extractions au phénol/chloroforme (1/1).

Le vecteur est le cosmide pHC79. Il est digéré de la même manière et déphosphorylé pour éviter toute autoligation.

La ligation s'effectue en mélangeant 700 ng de vecteur 0,8 µg de fragments d'ADN de 30/40 kb, le mélange est laissé à 14°C pendant 18 h après avoir ajouté 2,5 unités de T4 ADN ligase dans un tampon approprié.

Les cosmides recombinants sont encapsidés in vitro et utilisés pour transformer les bactéries (HB101). Les bactéries transformées sont incubées pendant 1h à 37°C en milieu LB puis étalées sur milieu sélectif Agar contenant 25 µg/ml d'Ampicilline. Les colonies résistantes à l'Ampicilline sont toutes testées pour leur sensibilité à la tétracycline, le fragment d'ADN de 30/40 kb ayant été inséré dans le vecteur de manière à inactiver le gène de résistance à la tétracycline (Tet) et à conserver le gène de résistance à l'ampicilline (Amp).

Il est effectué une mini-préparation d'ADN des 80 premières colonies transformantes résistantes à l'ampicilline (Amp^{r}) et sensibles à la tétracycline (Tet^{s}) selon la technique de la lyse alcaline. L'ADN de ces préparations est ensuite digéré par l'endonucléase de restriction SalI, analysé en electrophorèse sur gel d'agarose à 0,8 % puis transféré sur des filtres de nylon. L'ADN est fixé de façon irréversible par 5 mn d'exposition aux UV à 254 nm.

Ces différents filtres sont incubés pendant 16-18 heures à 68°C dans un tampon 6X SSC(1x SSC correspond à 0,15M Nacl et 0,015M citrate de Na) contenant 10% de sulfate de dextrane, une solution de Denhardt 5X concentrée (une solution de Denhardt 1X correspond à 0,02% de Ficoll, 0,02% de polyvinylpyrrolidone et 0,02% d'albumine sérique bovine), 10mM EDTA, 0,5% de SDS, 100 µg/ml d'ADN dénaturé de sperme de saumon et l'ADN génomique radiomarqué au phosphore 32 par multipriming, de l'une des trois espèces suivantes : M. avium, M. intracellulare, M. tuberculosis H37rv.

Après hybridation, les filtres sont lavés 2 fois pendant 10 mn en 2X SSC à 65°C, 1 fois pendant 30 mn en 2X SSC additionné de 0,1% SDS à 65°C et enfin 1 fois pendant 10 mn en 0,1X SSC à 65°C. Les filtres encore humides sont mis en autoradiographie à -80°C avec écran intensifiant de 1h à 2 jours.

Les résultats de ces hybridations ont permis d'isoler deux clones cosmidiques contenant des fragments d'environ 2kb nommés I6 et I1.

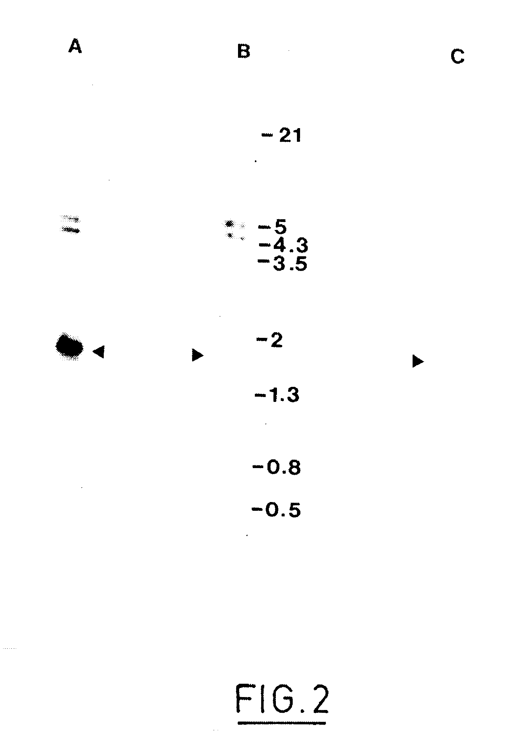

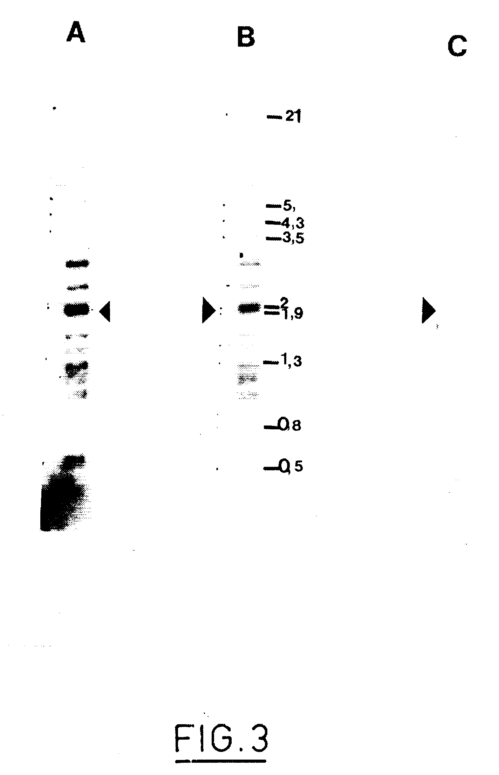

Le fragment I6 ne s'hybride qu'avec l'ADN génomique de M. avium, le fragment I1 s'hybride avec l'ADN de M. avium et de M. intracellulare. Ni l'un ni l'autre ne s'hybride avec l'ADN marqué de M. tuberculosis H37rv (figures 2 et 3).

Les fragments I6 et I1 ont été clonés dans un vecteur pUC18 et préparés en grande quantité. Les plasmides résultant ont été dénommés pMA01 et pMA02 respectivement.

Ces fragments ont été coupés par les enzymes SmaI, BamHI, PstI et EcoRI, puis clonés dans des phages M13mp18 et M13mp19 et séquencés selon la méthode de Sanger en utilisant la Taq polymérase en présence de d-azaGTP à la place du dGTP. Toutes les réactions de séquençage ont été réalisées avec du dATP marqué au ³⁵S.

Les schémas de la figure 8 représentent les stratégies suivies pour le séquençage des fragments I6 et I1.

Les séquences entières des fragments sont représentées en annexe (SEQ ID N°1 et SEQ ID N°2 correspondant respectivement au fragment I6 et au fragment I1). La composition entre l'ensemble de la banque de données de Los Alamos et les 1642 nucléotides du fragment I6 et les 2004 nucléotides du fragment I1, ainsi déterminés, ne fait ressortir aucune homologie significative avec les séquences connues aujourd'hui.

L'invention est illustrée par les exemples qui suivent :

### Exemple 1 :

Analyse d'ADN mycobactériens par la technique de Southern en utilisant comme sondes, les séquences d'acide nucléique de l'invention.

La liste des mycobactéries utilisées dans cette étude est donnée dans le tableau suivant :

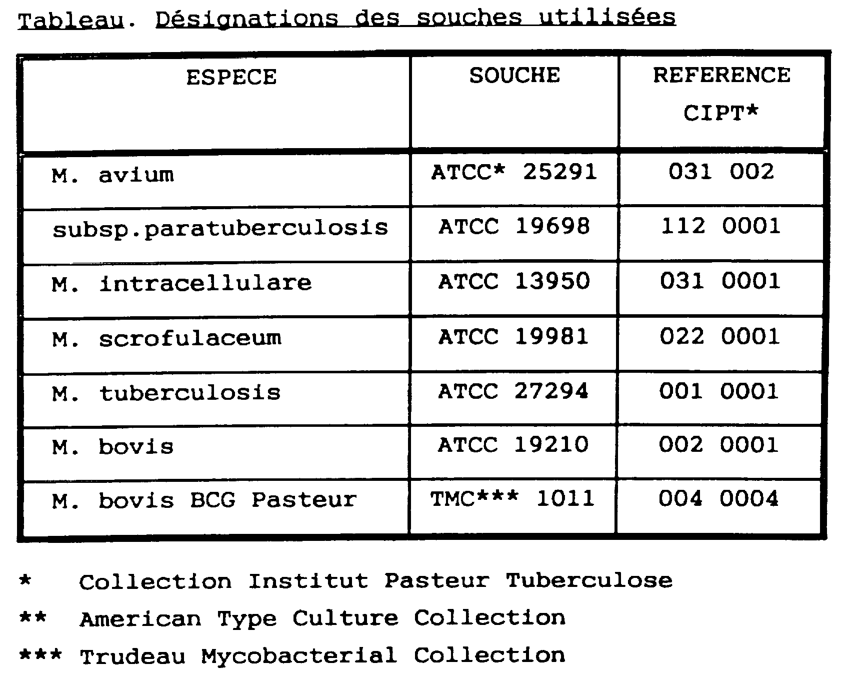

Après culture sur les milieux de Lowenstein-Jensen ou Middlebrook 7H9, les mycobactéries sont lysées en utilisant la technique suivante : les bactéries sont centrifugées pendant 30 minutes à 7000 g, resuspendues dans 20 ml de milieu 7H9 contenant 100 µg/ml de D-cyclosérine, 14 µg/ml de glycine, 200 µg/ml de lysozyme et 6 mM d'EDTA puis incubées à 37°C pendant 18 heures dans 4 ml de tampon de lyse constitué de Tris-HC1 50mM pH 8, d'EDTA 50 mM, de Nacl 100 mM, de SDS 0,5 % et contenant 60 µg/ml de pronase.

Après la lyse, les ADN sont extraits par le mélange phénol-chloroforme et précipités par l'éthanol. Les ADN ainsi extraits subissent une digestion totale par l'enzyme PstI. Les fragments obtenus sont ensuite séparés par électrophorèse sur gel d'agarose à 0,8% en TAE avant d'être transférés sur membrane de nitrocellulose selon la technique de Southern.

Les fragments transférés sont analysés par hybridation moléculaire. Dans cet exemple les sondes utilisées sont le fragment de 700 paires de bases extrait par digestion du fragment I6 par les enzymes SalI et EcoRI et pMA02 marqués par du phosphore 32 en suivant la technique du nécessaire "Multiprime" commercialisé par Amersham. Les résultats sont reportés aux figures 4 et 5.

La sonde issue de la séquence I6 détecte deux fragments avec les ADN des souches M. avium (sérotypes 1, 2, 3, 5, 6, 8, 9, 10, 11 et 21) et un seul fragment avec les ADN de la souche portant le sérotype 4. La taille de ces fragments varie avec la souche testée, différents profils sont ainsi obtenues en Southern blot (figure 4).

La sonde pMA02 contenant la séquence I1 détecte un fragment unique sur les ADN des souches M. intracellulare (sérotypes 2, 3, 7, 12, 13, 14, 15, 16, 17, 18, 19, 20, 23, 24 et 25). La taille du fragment obtenu est variable et est comprise entre 5 et 20 kb environ (figure 5).

### Exemple 2 :

Amplification enzymatique in vitro de l'ADN de souches M. avium-intracellulare avec les amorces définies à partir des séquences d'acide nucléique faisant l'objet de l'invention.

### Synthèse des amorces oligonucléotidiques

Les amorces dérivées des séquences I6 et I1 et ayant une longueur comprise entre 18 et 22 nucléotides ont été synthétisées sur un appareil automatisé Applied Biosystem basé sur la chimie des phosphoramidites. Après la synthèse, la solution d'oligonucléotide est transférée dans un tube de verre et incubée avec de l'hydroxyde d'ammonium concentré à 50°C pendant 4 heures. L'hydroxyde d'ammonium est ensuite évaporé dans un Speed Vac/Concentrator et le culot est lavé deux fois avec de l'eau distillée stérile et séché. Finalement, le culot est repris dans 0,5 ml de tampon Tris-HCl pH 8,0 contenant 1 mM d'EDTA. La concentration de chaque amorce est déterminéee au spectrophotomère et un aliquot est analysé en électrophorèse sur un gel de polyacrylamide pour vérifier que l'amorce n'est pas dégradée.

### Isolement de l'ADN mycobactérien

Les extraits biologiques sont traités de manière appropriée puis centrifugés. L'ADN est extrait par remise en suspension du culot avec 50 µl de NaOH 0,1 M contenant du NaCl 2M et du SDS 0,5%. Le mélange est incubé à 95°C 15 minutes, au mélange réactionnel on ajoute 400 µl de Tris-HCl 0,1M pH7. L'ADN est extrait 3 fois au phénol/chloroforme, précipité à l'éthanol et repris dans 50 µl de tampon Tris 10 mM pH8 contenant de l'EDTA 0,1mM.

### Amplification

La technique d'amplification enzymatique in vitro (PCR) est réalisée selon le protocole décrit par Saiki et al. (Science, 1988, 239, 487-491) en utilisant 12,5 pmoles des oligonucléotides du couple d'amorces n° 1 de la figure 1 d'une part ou MA3 et MA4 d'autre part et 5ng d'ADN de différentes souches de mycobactéries avec 2 unités de Taq polymérase dans un tampon contenant 50mM de KCl, 10mM de Tris-HCl pH 8,3, 2 mM de MgCl₂, 300 pH de désoxyribonucléotides triphosphates et 100 µg/ml de gélatine, le volume final de la réaction étant de 100 µl. Les paramètres des étapes de la PCR ont été choisis de la façon suivante : 2 mn à 94°C, 2 mn à 50°C, 2 mn à 72°C. Trente cycles sont réalisés en utilisant un appareil automatique. Après le dernier cycle les échantillons sont maintenus à 4°C jusqu'à l'analyse.

### Analyse électrophorétique sur un gel d'agarose des échantillons amplifiés.

Dix µl des échantillons sont déposés sur un gel d'agarose à 2% dans un tampon TAE (0,04 M Tris-acétate, 0,001M EDTA) contenant 1 µg/ml de bromure d'éthidium. Les fragments amplifiées sont visualisés sous UV et les gels sont photographiés en utilisant un film Polaroid 667.

Les figures 6 et 7 montrent les résultats obtenus avec différents ADN de mycobactéries et le couple d'amorces n° 1 d'une part (figure 6), et avec les amorces MA3 et MA4 d'autre part (figure 7).

Lorsque l'on utilise le couple d'amorce n° 1, un fragment d'ADN correspondant à la taille attendue est observé avec les ADN des mycobactéries suivantes : M. avium (sérotypes 1, 2, 3, 4, 5, 6, 8, 9, 10, 11 et 21) et M. paratuberculosis.

Il est important de noter que le couple d'amorce n°1 permet de détecter M. paratuberculosis rendant la méthode utilisable dans le domaine de la médecine vétérinaire. En effet, M. paratuberculosis est responsable de la maladie de Johne chez les ruminants. Cette maladie est une menace économique, elle a pour conséquence une perte de production de viande et de lait dont le coût est estimé, aux Etats Unis d'Amérique, à un milliard de dollars par an.

Lorsque l'on utilise le couple d'amorces MA3 et MA4, un fragment d'ADN correspondant à la taille attendue est observé avec les ADN des mycobactéries suivantes : M. avium (sérotypes 2, 3, 7, 12, 13, 14, 15, 16, 17, 18, 19, 20, 23, 24 et 25).

Par contre aucun fragment amplifié n'est visible lorsque l'ADN analysé est extrait des souches suivantes : M. tuberculosis, M. bovis, M. bovis-BCG, M. microti, M. scrofulaceum, M. fortuitum, M. kansasii et lorsque l'un des couples d'amorces n° 1, le couple MA1 et MA2 ou le couple MA3 et MA4 est utilisé pour diriger l'amplification.

### Liste des séquences

### I - INFORMATION GENERALE

(1) DEMANDEUR : INSTITUT PASTEUR
(2) TITRE DE L'INVENTION :
   SEQUENCES NUCLEOTIDIQUES HYBRIDANT SPECIFIQUEMENT AVEC LES SOUCHES BACTERIENNES DU COMPLEXE MYCOBACTERIUM AVIUM-INTRACELLULARE, LEUR UTILISATION COMME AMORCES OLIGONUCLEOTIDIQUES, SONDES NUCLEIQUES, METHODE D'IDENTIFICATION DES MYCOBACTERIES APPARTENANT AU COMPLEXE MYCOBACTERIUM AVIUM-INTRACELLULARE, NECESSAIRE POUR LA MISE EN OEUVRE DE LA METHODE.
(3) NOMBRE DE SEQUENCES : 2

### II - INFORMATION POUR SEQ ID N°1

### CARACTERISTIQUES DES SEQUENCES

- TYPE: : Nucléotide
- LONGUEUR: : 1642 paires de bases
- NOMBRE DE BRINS: : simple
- CONFIGURATION: : linéaire
- TYPE DE MOLECULE: : ADN
- ORGANISME: : Mycobacterium avium
- NOM: : I-6

### DESCRIPTION DE LA SEQUENCE

10                  20                  30                  40                  50
GACCAGTTCCGCGTGCATTCGCCCGCCCACCTCGCCGACCAGCGAGGCCG
60                  70                  80                  90                  100
TGTGGTCGAGATCGTCGGGTGTGCTGGCGGAACCATCACCGTGTCCACC
110                 120                 130                 140                 150
GGTCCGCACAGGTCGAGGGCCACGATGTCGGCGCCCTCCTGCGCGAACCG

160 170 180 190 200
CACGGCTTGGCGCGCCCGATTCCCCGCGCCGCGCCCGTGATCAACGCGA
210 220 230 240 250
CCTTCCCCGCCAGCCGCCCCGGACTGACTTGTGGCATCCGGCGATTCTGC
260 270 280 290 300
CGCGCGAGCGGCACCGGGGTCGGTGTGTGTCCGATGGCCGGAGACGATC
310 320 330 340 350
TATGCCGGCGTACCGGTCACCGGATATCCCGGCCGAGGGCGACCGCCGG
360 370 380 390 400
GACCTAACGTAGGCATCCGTGACCAGTGGCAGTGACCGGCGCGCAACCCC
410 420 430 440 450
GGACGTCGATGTGGTGGTCGTCGGCGCGGCTTCGCCGGCCTGTACGCGCT
460 470 480 490 500
GCACAAACTGCGATCGAACGGCTGCGGGTCAGGGTGTTCGAGGCCGGGCC
510 520 530 540 550
GGACGTCGGCGGAACCTGGTACTTCAACCGCTACCCCGGGGCCCGATGCG
560 570 580 590 600
ACGTCGAGAGCGTGGACTACTGCTACTCGTTCTCCGACGCGCTGCAGCGG
610 620 630 640 650
GAGTGGATTGGTCGGAGAAGTACGCCACCCAACCCGAGATTCTGGCCTA
660 670 680 690 700
CATCAATTGGGTCGCCGACCGGCTGGATCTGCGCCGCGACATCACCCTGA
710 720 730 740 750
ACGCCCGCGTGAATTCCGCCGTGCTCGACGAAGCGCAGTTGCGCTGGACG
760 770 780 790 800
GTGACCACCGAGACGGGCGAGCGCGTCACCGCCCGGTTCTGCGTGATGGC
810 820 830 840 850
CACCGGCCCGTTGTCGGCGGCGATGACTCCGCCGTTCCCCGGTTTGGATA
860 870 880 890 900
CCTTCGCCGGGCAGGTCTACCACACCGCGGCCTGGCCGCACGAACGGTGG
910 920 930 940 950
ACTTCACCGGCAAGCGCGTCGCCGTCATTGGCACGGATCCCCGGGTCTG
960 970 980 990 1000
GCATCCAATGCAGATTCGCGATCACTCGACCGAGGCCGCATCGCAGCTGT
1010 1020 1030 1040 1050
ACGTGTTCCAGCGCACCCCGAATTACAGTGTGCCGGCGGGTAACCGGCCG
1060 1070 1080 1090 1100
TTGAGTGACTCCGACCGTGCCGAGGTGAAGGCCCATTACGCGGAACGACG
1110 1120 1130 1140 1150
ACGGATATCGTGGCGCACGGGGGGCGGCTCGCCCACGTCGCCCACCCGAA
1160 1170 1180 1190 1200
GTTGACGATGGAGGCAACCCCCGAGGAACGGCGTGAGGCATTCGAAAAGC

1210 1220 1230 1240 1250
GCTGGGAGCTCGGCGAGTGCTGTTCTCCAAGACATTCGCCGACCAGATGA
1260 1270 1280 1290 1300
TCGACCCGGTCGCCAACGAGGAGGCCCGCAAGTTCTACGAGGAGAAGGTG
1310 1320 1330 1340 1350
CGCGCGGTCATCGACGACCCGGTGCTGGCCGACCTGCTCATCCCGAATGA
1360 1370 1380 1390 1400
CCATCCGATCGGCACCAAGCGAATCTGCACCGACAGCAACTACTTTCAGA
1410 1420 1430 1440 1450
CCTTCAACCGCCCGAACGTGAAGCTGATCAGCGTCCGCAAGACGCCGATC
1460 1470 1480 1490 1500
ATGTCCATCGATGCGACCGGCATTAACACCACCGACGCCCATTACGACCT
1510 1520 1530 1540 1550
CGACGCGATCGTGCTGGCGACCGGATTCGACGCGATGACCGGCGCGCTGG
1560 1570 1580 1590 1600
CCAAGATCGACATCGTGGGCCGCGACGGAAGGCGGCTGAGCGACGACTGG
1610 1620 1630 1640
TCCGGCGGGCCCCGCACGTACCTGGGCCTCGGCGTCGACCTG

### III - INFORMATION POUR SEQ ID N°2

### CARACTERISTIQUES DES SEQUENCES

- TYPE: : Nucléotide
- LONGUEUR: : 2004 paires de bases
- NOMBRE DE BRINS: : simple
- CONFIGURATION: : linéaire
- TYPE DE MOLECULE: : ADN
- ORGANISME: : Mycobacterium avium
- NOM: : I-1

### DESCRIPTION DE LA SEQUENCE

10 20 30 40 50
GTCGACGCCACCACACTGCCCCACGACATCGAACGTCCTGGCCGGCACGA
60 70 80 90 100
TCGCGAAGGCGGGAACGGTTGTCGGCAGCGAATTCTCGTGGGTCGGCCA
110 120 130 140 150
CTGGTCGGGAACGCCCGTTGGCTGGCCATTCACGAAGGAGTGGGTGCTCA
160 170 180 190 200
CCCGCGAACCTTCCACAATGGGGCATGGCTCCATTGGCGCCCGGCGAAAA
210 220 230 240 250
GGACGCCGCTGATCCGCGCGGTCATCAAAGGTGAGCCCAGCTTTGAACTC
260 270 280 290 300
CAGCTCGACGTGGCATTCGACGGCGCGCCATCGAACGGGCCAGCACGCCA
310 320 330 340 350
TGCCAGGTCACCTGATGATCGCGAATGAAGCGCGGTTCGCGCCATACCGT
360 370 380 390 400
ACGTGCTGGCCCGGCCACCCGGTGTCGTGACAGCACCGGTGTTCGGCGCG
410 420 430 440 450
ATCCAACTAGCCTGAGGCACCACCGACCGCGGGCGATGTGGTTCGCTG
460 470 480 490 500
GGCGCCGCATGGAAAACGTGCGCGCTGCCGTCGGGCAAAACCTTCGGCC
510 520 530 540 550
ACGAGATTAATCGGAACCCATCCACCCCTGTCGGATGAACCGGTCCGAAT
560 570 580 590 600
TCGCAGGTAACGTTCCCGGCGCGCCTGCTGGCCGACGGAACGAGCCTTT
610 620 630 640 650
CACCTGCTCCATTCCCGTTCTTCACACCCTCCCCGGTTCAACGGCCGTGC
660 670 680 690 700
CGCGGCGAGACCACGCACGATCACGGTGGCCGCGTCGTGCGACAGGCCCG
710 720 730 740 750
GCATCGAGTGTCCGGGCCGGCGACCGTATCGCGCCTCGAAGCGGTCGAGG
760 770 780 790 800
AAGGCCTGTCCGACCGTGTTGCGCTCGTCGTAGCTGTCCAGGCCGATCCA

810 820 830 840 850
TCCGGATAGGTGCCGCCTCCACTCCGCGCTGATGTGTGCCATTTCGAACG
860 870 880 890 900
CCGTCGTCGTGTATCGCGGCGGATCCCAGCCCGCGGCGCAGCGCATCC
910 920 930 940 950
GTGAAGCCCCACAGCCCGTGCCCGAACCCGAACGTGCACCAGCCGTCGGG
960 970 980 990 1000
TTCCGCCGCACGCAACGCAGTGACCGTCTCGGACTTGTCGGCTTCGACCT
1010 1020 1030 1040 1050
GGGGAATCGCCACGGTCGCAACGACCTTCAAACCCGCGGCGTCGTAGGCT
1060 1070 1080 1090 1100
CTCTCGGCGAAGGCGAGGTACCTCCTTACCGATGAGCGATGCCTCGTAGC
1110 1120 1130 1140 1150
AATGCGATGCGCGGATCGACCGTCGCCGGATCATCACCGCGGCCAGCATC
1160 1170 1180 1190 1200
ACCGGCTCCTCGGGCATCGAGCCGTTGTTCAGTCGAAGCACCAGTCGCCG
1210 1220 1230 1240 1250
AGCGCTCCCTCCGATCCCGACAGAGTGACGATCGGAACCCGGCGCGGTGG
1260 1270 1280 1290 1300
CCTCGACGTGGGAACGCAGCGGCACCACGTTATCCGACACCCACGGGCCC
1310 1320 1330 1340 1350
GAAGATCGCCAGGCAACCCTCCGCGACGAGCTCGTCGAAGGCCCGCTCCG
1360 1370 1380 1390 1400
ACCACTTGGTAGGTCCCGTTGGGCAGGCCCACCACATTGCGGGTGACGAG
1410 1420 1430 1440 1450
TTCGATCGGCCGGTCGATCAGTCCCGAGGAAAGGGCTTCATCCATCACCA
1460 1470 1480 1490 1500
GCCGCAGCGCCGACGCTGTCGTTGTCGGTGTCGCCGGACGTCGGATAGTC
1510 1520 1530 1540 1550
GTTGAGCAAGCCGACTTTCAGGGGAGCGACCGAACCGTATGCGCGAACCT
1560 1570 1580 1590 1600
CGTCTGCCATAGCGGCAACATACAGATATGGAGGTTGAGGCCCACCGCGC
1610 1620 1630 1640 1650
CACCGCAAACTCGCACCCGCCGAAAATGGTGGTTTACGAAAAGTGCAAAG
1660 1670 1680 1690 1700
CGAACTTTCGGCATGTTAGTTTTTCCGAGCGGGGGATCACATTCCGGAGG
1710 1720 1730 1740 1750
AGACGGAATGAACGCTCACATCGACGCAGGAGTCCCACGCGCCGTCAATC
1760 1770 1780 1790 1800
CAGAAGAAGTACCGCGTGATCCAGTGGGGCATGGGTAATGTCGGCACGAT
1810 1820 1830 1840 1850
CGCACTGCGCCACTTCGCACACAACCCTCTCTACGAAGTGGTCGGAGTGC
1860 1870 1880 1890 1900
TTTGTAATCGCCCGGAGAAGGTCGGTAGGGAACGCCGGTGAACTTCGTCG

1910        1920        1930        1940        1950
GGGTAGGACCGGATCGGGGTACACGCAACCACCGATAAGGCCACCTTGGA
1960        1970        1980        1990        2000
GACACTCGACAGCCGATTGCGTGTTCTACGCGCCGCTCTGGTCCGGACGT

CGAC

### IV - INFORMATION POUR LES PAIRES DE SEQUENCES NUCLEOTIDIQUES N°1 A N°28 CONSTITUANT DES AMORCES (SEQ ID N°3 A SEQ ID N°58) PROVENANT DE LA SEQUENCE SEQ ID N°1

### CARACTERISTIQUES DES SEQUENCES

- TYPE: : Nucléotides
- LONGUEUR: : 30 bases
- NOMBRE DE BRINS: : simple
- CONFIGURATION: : linéaire
- TYPE DE MOLECULE: : ADN
- ORGANISME: : Mycobacterium-avium

### DESCRIPTION DES SEQUENCES

couple d'amorces n°1 :

SEQ ID N°3 : 5' ATGGCCGGAGACGATCTATGCCGGCGTAC 3'

SEQ ID N°4 : 5' CGTTCGATCGCAGTTTGTGCAGCGCGTACA 3'

couple d'amorces n°2 :

SEQ ID N°5 : 5' ATGGCCGGAGACGATCTATGCCGGCGTAC 3'

SEQ ID N°6 : 5' AACACCCTGACCCGCAGCCGTTCGATCGCA 3'

couple d'amorces n°3 :

SEQ ID N°7 : 5' ATGGCCGGAGACGATCTATGCCGGCGTAC 3'

SEQ ID N°8 : 5' GAACACCCTGACCCGCAGCCGTTCGATCGC 3'

couple d'amorces n°4 :

SEQ ID N°9 : 5' ATGGCCGGAGACGATCTATGCCGGCGTAC 3'

SEQ ID N°10 : 5' CGAACACCCTGACCCGCAGCCGTTCGATCG 3'

couple d'amorces n°5 :

SEQ ID N°11 : 5' GCCGGGAGACGATCTATGCCGGCGTACCGG 3'

SEQ ID N°12 : 5' CGTTCGATCGCAGTTTGTGCAGCGCGTACA 3'

couple d'amorces n°6 :

SEQ ID N°13 : 5' GCCGGGAGACGATCTATGCCGGCGTACCGG 3'

SEQ ID N°14 : 5' AACACCCTGACCCGCAGCCGTTCGATCGCA 3'

couple d'amorces n°7 :

SEQ ID N°15 : 5' GCCGGGAGACGATCTATGCCGGCGTACCGG 3'

SEQ ID N°16 : 5' GAACACCCTGACCCGCAGCCGTTCGATCGC 3'

couple d'amorces n°8 :

SEQ ID N°17 : 5' GCCGGGAGACGATCTATGCCGGCGTACCGG 3'

SEQ ID N°18 : 5' CGAACACCCTGACCCGCAGCCGTTCGATCG 3'

couple d'amorces n°9 :

SEQ ID N°19 : 5' CCGGCCGAGGGCGACCGCCGGGACCTAACG 3'

SEQ ID N°20 : 5' CGTTCGATCGCAGTTTGTGCAGCGCGTACA 3'

couple d'amorces n°10 :

SEQ ID N°21 : 5' CCGGCCGAGGGCGACCGCCGGGACCTAACG 3'

SEQ ID N°22 : 5' AACACCCTGACCCGCAGCCGTTCGATCCGA 3'

couple d'amorces n°11 :

SEQ ID N°23 : 5' CCGGCCGAGGGCGACCGCCGGACCTAACG 3'

SEQ ID N°24 : 5' GAACACCCTGACCCGCAGCCGTTCGATCGC 3'

couple d'amorces n°12 :

SEQ ID N°25 : 5' CCGGCCGAGGGCGACCGCCGGACCTAACG 3'

SEQ ID N°26 : 5' CGAACACCCTGACCCGCAGCCGTTCGATGC 3'

couple d'amorces n°13 :

SEQ ID N°27 : 5' TCAACCGCTACCCCGGGGCCCGATGCGACG 3'

SEQ ID N°28 : 5' TCACCGTCCAGCGCAACTGCGCTTCGTCGA 3'

couple d'amorces n°14 :

SEQ ID N°29 : 5' GCTACCCCGGGGCCCGATGCGACGTCGAGA 3'

SEQ ID N°30 : 5' TCACCGTCCAGCGCAACTGCGCTTCGTCGA 3'

couple d'amorces n°15 :

SEQ ID N°31 : 5' GCCCGCGTGAATTCCGCCGTGCTCGACGAA 3'

SEQ ID N°32 : 5' GACGCGCTTGCCGGTGAAGTCCACCGTTCG 3'

couple d'amorces n°16 :

SEQ ID N°33 : 5' GCCCGCGTGAATTCCGCCGTGCTCGACGAA 3'

SEQ ID N°34 : 5' GGATCCCGTGCCAATGACGGCGACGCGCTT 3'

couple d'amorces n°17 :

SEQ ID N°35 : 5' GCCCGCGTGAATTCCGCCGTGCTCGACGAA 3'

SEQ ID N°36 : 5' GATGCGGCCTCGGTCGAGTGATCGCGAATC 3'

couple d'amorces n°18 :

SEQ ID N°37 : 5' GCCCGCGTGAATTCCGCCGTGCTCGACGAA 3'

SEQ ID N°38 : 5' CGATGCGGCCTCGGTCGAGTGATCGCGAAT 3'

couple d'amorces n°19 :

SEQ ID N°39 : 5' GCCCGCGTGAATTCCGCCGTGCTCGACGAA 3'

SEQ ID N°40 : 5' GCGATGCGGCCTCGGTCGAGTGATCGCGAA 3'

couple d'amorces n°20 :

SEQ ID N°41 : 5' GCCCGCGTGAATTCCGCCGTGCTCGACGAA 3'

SEQ ID N°42 : 5' TGCGATGCGGCCTCGGTCGAGTGATCGCGA 3'

couple d'amorces n°21 :

SEQ ID N°43 : 5' GCCCGCGTGAATTCCGCCGTGCTCGACGAA 3'

SEQ ID N°44 : 5' CACGTACAGCTGCGATGCGGCCTCGGTCGA 3'

couple d'amorces n°22 :

SEQ ID N°45 : 5' ACCACACCGCGGCCTGGCCGCACGAACGGT 3'

SEQ ID N°46 : 5' GCCACGATATCCGTCGTCGTTCCGCGTAAT 3'

couple d'amorces n°23 :

SEQ ID N°47 : 5' ACGAACGGTGGACTTCACCGGCAACGCCGT 3'

SEQ ID N°48 : 5' GCCACGATATCCGTCGTCGTTCCGCGTAAT 3'

couple d'amorces n°24 :

SEQ ID N°49 : 5' CCCGGGTCTGGCATCCAATCGAGATTCGCG 3'

SEQ ID N°50 : 5' GCCACGATATCCGTCGTCGTTCCGCGTAAT 3'

couple d'amorces n°25 :

SEQ ID N°51 : 5' CCGGGTCTGGCATCCAATGCAGATTCGCGA 3'

SEQ ID N°52 : 5' GCCACGATATCCGTCGTCGTTCCGCGTAAT 3'

couple d'amorces n°26 :

SEQ ID N°53 : 5' CGGGTCTGGCATCCAATGCAGATTCGCGAT 3'

SEQ ID N°54 : 5' GCCACGATATCCGTCGTCGTTCCGCGTAAT 3'

couple d'amorces n°27 :

SEQ ID N°55 : 5' GGGTCTGGCATCCAATGCAGATTCGCGATC 3'

SEQ ID N°56 : 5' GCCACGATATCCGTCGTCGTTCCGCGTAAT 3'

couple d'amorces n°28 :

SEQ ID N°57 : 5' TCCAATGCAGATTCGCGATCACTCGACCGA 3'

SEQ ID N°58 : 5' GCCACGATATCCGTCGTCGTTCCGCGTAAT 3'

### V - INFORMATION POUR LES PAIRES DE SEQUENCES NUCLEOTIDIQUES N°29 A N°79 CONSTITUANT DES AMORCES (SEQ ID N°59 A SEQ ID N°160) PROVENANT DE LA SEQUENCE SEQ ID N°2

### CARACTERISTIQUES DES SEQUENCES

- TYPE: : Nucléotide
- LONGUEUR: : 30 bases
- NOMBRE DE BRINS: : simple
- CONFIGURATION: : linéaire
- TYPE DE MOLECULE: : ADN
- ORGANISME: : Mycobacterium-avium

### DESCRIPTION DES SEQUENCES

Couple d'amorces n°29:

SEQ ID N°59 : 5' CGGCATCGAGTGTCCGGGCCGGCGACCGTA 3'

SEQ ID N°60 : 5' ATCCGCCGCGATACACGACGACGGCGTTCG 3'

Couple d'amorces n°30:

SEQ ID N°61 : 5' CGGCATCGAGTGTCCGGGCCGGCGACCGTA 3'

SEQ ID N°62 : 5' GCGCCGCGGGCTGGGATCCGCCGCGATACA 3'

Couple d'amorces n°31:

SEQ ID N°63 : 5' CGGCATCGAGTGTCCGGGCCGGCGACCGTA 3'

SEQ ID N°64 : 5' CGCGCCGCGGGCTGGGATCCGCCGCGATAC 3'

Couple d'amorces n°32:

SEQ ID N°65 : 5' CGGCATCGAGTGTCCGGGCCGGCGACCGTA 3'

SEQ ID N°66 : 5' GCGCGCCGCGGGCTGGGATCCGCCGCGATA 3'

Couple d'amorces n° 33:

SEQ ID N°67 : 5' CATCGAGTGTCCGGCCGGCGACCGTATCG 3'

SEQ ID N°68 : 5' ATCCGCCGCGATACACGACGACGGCGTTCG 3'

Couple d'amorces n° 34:

SEQ ID N°69 : 5' CATCGAGTGTCCGGCCGGCGACCGTATCG 3'

SEQ ID N°70 : 5' GCGCCGCGGGCTGGATCCGCCGCGATACA 3'

Couple d'amorces n° 35:

SEQ ID N°71 : 5' CATCGAGTGTCCGGCCGGCGACCGTATCG 3'

SEQ ID N°72 : 5' CGCGCCGCGGGCTGGATCCGCCGCGATAC 3'

Couple d'amorces n° 36:

SEQ ID N°73 : 5' CATCGAGTGTCCGGCCGGCGACCGTATCG 3'

SEQ ID N°74 : 5' GCGCGCCGCGGGCTGGATCCGCCGCGATA 3'

Couple d'amorces n° 37:

SEQ ID N°75 : 5' ATCGAGTGTCCGGCCGGCGACCGTATCGC 3'

SEQ ID N°76 : 5' ATCCGCCGCGATACACGACGACGGCGTTCG 3'

Couple d'amorces n° 38 :

SEQ ID N°77 : 5' ATCGAGTGTCCGGCCGGCGACCGTATCGC 3'

SEQ ID N°78 : 5' GCGCCGCGGGCTGGATCCGCCGCGATACA 3'

Couple d'amorces n° 39 :

SEQ ID N°79 : 5' ATCGAGTGTCCGGGCCGGCGACCGTATCGC 3'

SEQ ID N°80 : 5' CGCGCCGCGGGCTGGATCCGCCGCGATAC 3'

Couple d'amorces n° 40 :

SEQ ID N°81 : 5' ATCGAGTGTCCGGGCCGGCGACCGTATCGC 3'

SEQ ID N°82 : 5' GCGCGCCGCGGGCTGGATCCGCCGCGATA 3'

Couple d'amorces n° 41 :

SEQ ID N°83 : 5' TCGAGTGTCCGGGCCGGCGACCGTATCGCG 3'

SEQ ID N°84 : 5' ATCCGCCGCGATACACGACGACGGCGTTCG 3'

Couple d'amorces n° 42 :

SEQ ID N°85 : 5' TCGAGTGTCCGGGCCGGCGACCGTATCGCG 3'

SEQ ID N°86 : 5' GCGCCGCGGGCTGGATCCGCCGCGATACA 3'

Couple d'amorces n° 43 :

SEQ ID N°87 : 5' TCGAGTGTCCGGGCCGGCGACCGTATCGCG 3'

SEQ ID N°88 : 5' CGCGCCGCGGGCTGGATCCGCCGCGATAC 3'

Couple d'amorces n° 44 :

SEQ ID N°89 : 5' TCGAGTGTCCGGGCCGGCGACCGTATCGCG 3'

SEQ ID N°90 : 5' GCGCGCCGCGGGCTGGATCCGCCGCGATA 3'

Couple d'amorces n° 45 :

SEQ ID N°91 : 5' CGAGTGTCCGGCCGGCGACCGTATCGGCG 3'

SEQ ID N°92 : 5' ATCCGCCGCGATACACGACGACGGCGTTCG 3'

Couple d'amorces n° 46 :

SEQ ID N°93 : 5' CGAGTGTCCGGCCGGCGACCGTATCGGCG 3'

SEQ ID N°94 : 5' GCGCCGCGGGCTGGGATCCGCCGCGATACA 3'

Couple d'amorces n° 47 :

SEQ ID N°95 : 5' CGAGTGTCCGGCCGGCGACCGTATCGGCG 3'

SEQ ID N°96 : 5' CGCGCCGCGGGCTGGGATCCGCCGCGATAC 3'

Couple d'amorces n° 48 :

SEQ ID N°97 : 5' CGAGTGTCCGGCCGGCGACCGTATCGGCG 3'

SEQ ID N°98 : 5' GCGCGCCGCGGGCTGGGATCCGCCGCGATA 3'

Couple d'amorces n° 49 :

SEQ ID N°99 : 5' CGCAACGACCTTCAAACCCGCGGCGTCGTA 3'

SEQ ID N°100 5' TCGAGGCCACCGCGCCGGGTTCCGATCGTC 3'

Couple d'amorces n° 50 :

SEQ ID N°101 5' CGCAACGACCTTCAAACCCGCGGCGTCGTA 3'

SEQ ID N°102 5' GTCGAGGCCACCGCGCCGGGTTCCGATCGT 3'

Couple d'amorces n° 51 :

SEQ ID N°103 5' TGCTCTGTAGCAATGCGATGCGCGGATCGA 3'

SEQ ID N°104 5' TCGAGGCCACCGCGCCGGGTTCCGATCGTC 3'

Couple d'amorces n° 52 :

SEQ ID N°105 5' TGCTCTGTAGCAATGCGATGCGCGGATCGA 3'

SEQ ID N°106 5' GTCGAGGCCACCGCGCCGGGTTCCGATCGT 3'

Couple d'amorces n°53 :

SEQ ID N°107 :5' TCGTAGCAATGCGATGCGCGGATCGACCGT 3'

SEQ ID N°108 :5' TCGAGGCCACCGCGCCGGGTTCCGATCGTC 3'

Couple d'amorces n°54 :

SEQ ID N°109 :5' TCGTAGCAATGCGATGCGCGGATCGACCGT 3'

SEQ ID N°110 :5' GTCGAGGCCACCGCGCCGGGTTCCGATCGT 3'

Couple d'amorces n° 55:

SEQ ID N°111 :5' GTAGCAATGCGATGCGCGGATCGACCGTCG 3'

SEQ ID N°112 :5' TCGAGGCCACCGCGCCGGGTTCCGATCGTC 3'

Couple d'amorces n°56 :

SEQ ID N°113 :5' GTAGCAATGCGATGCGCGGATCGACCGTCG 3'

SEQ ID N°114 :5' GTCGAGGCCACCGCGCCGGGTTCCGATCGT 3'

Couple d'amorces n°57 :

SEQ ID N°115 :5' CCACCACATTGCGGGTGACGAGTTCGATCG 3'

SEQ ID N°116 5' TCCCCTGAAAGTCGGCTTGCTCAACGACTA 3'

Couple d'amorces n°58 :

SEQ ID N°117 5' CCACCACATTGCGGGTGACGAGTTCGATCG 3'

SEQ ID N°118 5' TATGGCAGACGAGGTTCGCGCATACGGTTC 3'

Couple d'amorces n° 59 :

SEQ ID N°119 : 5' CCACCACATTGCGGGTGACGAGTTCGATCG 3'

SEQ ID N°120 : 5' CTATGGCAGACGAGGTTCGCGCATACGGTT 3'

Couple d'amorces n° 60 :

SEQ ID N°121 : 5' CCACCACATTGCGGGTGACGAGTTCGATCG 3'

SEQ ID N°122 : 5' CCGCTATGGCAGACGAGGTTCGCGCATACG 3'

Couple d'amorces n°61 :

SEQ ID N°123 : 5' CCACCACATTGCGGGTGACGAGTTCGATCG 3'

SEQ ID N°124 : 5' TGCCGCTATGGCAGACGAGGTTCGCGCATA 3'

Couple d'amorces n° 62 :

SEQ ID N°125 : 5' CACCACATTGCGGGTGACGAGTTCGATCGG 3'

SEQ ID N°126 : 5' TCCCCTGAAAGTCGGCTTGCTCAACGACTA 3'

Couple d'amorces n° 63 :

SEQ ID N°127 :5' CACCACATTGCGGGTGACGAGTTCGATCGG 3'

SEQ ID N°128 :5' TATGGCAGACGAGGTTCGCGCATACGGTTC 3'

Couple d'amorces n° 64 :

SEQ ID N°129 :5' CACCACATTGCGGGTGACGAGTTCGATCGG 3'

SEQ ID N°130 :5' CTATGGCAGACGAGGTTCGCGCATACGGTT 3'

Couple d'amorces n° 65 :

SEQ ID N°131 :5' CACCACATTGCGGGTGACGAGTTCGATCGG 3'

SEQ ID N°132 :5' CCGCTATGGCAGACGAGGTTCGCGCATACG 3'

Couple d'amorces n° 66 :

SEQ ID N°133 :5' CACCACATTGCGGGTGACGAGTTCGATCGG 3'

SEQ ID N°134 :5' TGCCGCTATGGCAGACGAGGTTCGCGCATA 3'

Couple d'amorces n° 67 :

SEQ ID N°135 :5' CTGTCGTTGTCGGTGTCGCCGGACGTCGAA 3'

SEQ ID N°136 :5' GATTGACGGCGCGTGGGACTCCTGCGTCGA 3'

Couple d'amorces n° 68 :

SEQ ID N°137 :5' TGTCGTTGTCGGTGTCGCCGGACGTCGGAT 3'

SEQ ID N°138 :5' GATTGACGGCGCGTGGGACTCCTGCGTCGA 3'

Couple d'amorces n° 69 :

SEQ ID N°139 :5' GTCGGTGTCGCCGGACGTCGGATAGTCGTT 3'

SEQ ID N°140 :5' GATTGACGGCGCGTGGACTCCTGCGTCGA 3'

Couple d'amorces n° 70 :

SEQ ID N°141 :5' GCCGACTTTCAGGGGAGCGACCGAACCGTA 3'

SEQ ID N°142 :5' GATTGACGGCGCGTGGACTCCTGCGTCGA 3'

Couple d'amorces n°71 :

SEQ ID N°143 :5' GCCGACTTTCAGGGGAGCGACCGAACCGTA 3'

SEQ ID N°144 :5' AAGTGGCGCAGTGCGATCGTGCCGACATTA 3'

Couple d'amorces n° 72 :

SEQ ID N°145 :5' CCGACTTTCAGGGGAGCGACCGAACCGTAT 3'

SEQ ID N°146 :5' GATTGACGGCGCGTGGACTCCTGCGTCGA 3'

Couple d'amorces n°73 :

SEQ ID N°147 :5' CCGACTTTCAGGGGAGCGACCGAACCGTAT 3'

SEQ ID N°148 :5' AAGTGGCGCAGTGCGATCGTGCCGACATTA 3'

Couple d'amorces n°74 :

SEQ ID N°149 :5' ACTTTCAGGGGAGCGACCGAACCGTATGCG 3'

SEQ ID N°150 :5' GATTGACGGCGCGTGGACTCCTGCGTCGA 3'

Couple d'amorces n° 75 :

SEQ ID N°151 : 5' ACTTTCAGGGGAGCGACCGAACCGTATGCG 3'

SEQ ID N°152 : 5' AAGTGGCGCAGTGCGATCGTGCCGACATTA 3'

Couple d'amorces n°76 :

SEQ ID N°153 : 5' TTTCAGGGGAGCGACCGAACCGTATGCGCG 3'

SEQ ID N°154 : 5' GATTGACGGCGCGTGGGACTCCTGCGTCGA 3'

Couple d'amorces n°77 :

SEQ ID N°155 : 5' TTTCAGGGGAGCGACCGAACCGTATGCGCG 3'

SEQ ID N°156 : 5' AAGTGGCGCAGTGCGATCGTGCCGACATTA 3'

Couple d'amorces n°78 :

SEQ ID N°157 : 5' GGAGACGGAATGAACGCTCACATCGACGCA 3'

SEQ ID N°158 : 5' CGTTCCCTACCGACCTTCTCCGGGCGATTA 3'

Couple d'amorces n°79 :

SEQ ID N°159 : 5' GGAGACGGAATGAACGCTCACATCGACGCA 3'

SEQ ID N°160 : 5' TTATCGGTGGTTGCGTGTACCCCGATCCGG 3'

### VI - INFORMATION POUR LA PAIRE DE SEQUENCES NUCLEOTIDIQUES N°80 CONSTITUANT UNE AMORCE (SEQ ID N°161 ET SEQ ID N°162) PROVENANT DE LA SEQUENCE SEQ ID N°1

### CARACTERISTIQUES DES SEQUENCES

- TYPE: : Nucléotide
- LONGUEUR: : 20 bases
- NOMBRE DE BRINS: : simple
- CONFIGURATION: : linéaire
- TYPE DE MOLECULE: : ADN
- ORGANISME: : Mycobacterium-avium
- NOM: : MA1 - MA2

### DESCRIPTION DES SEQUENCES

SEQ ID N°161 : 5'GGC CCG ATG CGA CGT CGA GA3'
SEQ ID N°162 : 5'GCG CAA CTG CGC TTC GTC GA3'

### VII - INFORMATION POUR LA PAIRE DE SEQUENCES NUCLEOTIDIQUES N°81 CONSTITUANT UNE AMORCE (SEQ ID N°163 ET SEQ ID N°164) PROVENANT DE LA SEQUENCE SEQ ID N°2

### CARACTERISTIQUE DES SEQUENCES

- TYPE: : Nucléotide
- LONGUEUR: : 30 bases
- NOMBRE DE BRINS: : simple
- CONFIGURATION: : linéaire
- TYPE DE MOLECULE: : ADN
- ORGANISME: : Mycobacterium-avium
- NOM: : MA3 - MA4

### DESCRIPTION DES SEQUENCES

SEQ ID N°163 : 5'GAA CGC CCG TTG CTG GCC ATT CAC GAG GAG3'
SEQ ID N°164 : 5'GCG CAA CAC GGT CGG ACA GGC CTT CCT CGA3'

## Revendications

1. Séquence d'acide nucléique qui hybride spécifiquement avec une séquence d'acide nucléique génomique des souches appartenant au complexe Mycobacterium avium-intracellulare choisie parmi la séquence nucléotidique SEQ ID N°1, la séquence nucléotidique SEQ ID N°2, les séquences complémentaires de celles-ci, ainsi que les séquences qui en diffèrent par mutation, insertion, délétion ou substitution d'une ou plusieurs bases, et qui n'hybride pas avec les acides nucléiques des mycobactéries n'appartenant pas audit complexe.

2. Séquence d'acide nucléique qui hybride spécifiquement avec l'acide nucléique génomique des souches appartenant au complexe M. avium-intracellulare portant les sérotypes 2, 3, 7, 12, 13, 14, 15, 16, 17, 18, 19, 20, 23, 24 et 25, et qui n'hybride pas avec les acides nucléiques des mycobactéries n'appartenant pas audit complexe, caractérisée en ce qu'elle est constituée par la séquence nucléotidique SEQ ID N°2, selon la revendication 1, une séquence complémentaire de celle-ci, ainsi que les séquences qui en diffèrent par mutation, insertion, déletion ou substitution d'une ou plusieurs bases.

3. Séquence d'acide nucléique qui hybide spécifiquement avec une séquence d'acide nucléique génomique des souches appartenant à M.avium portant le sérotype 1, 2, 3, 4, 5, 6, 8, 9, 10, 11 ou 21 caractérisée en ce qu'elle est constituée d'un fragment d'acide nucléique de 700 paires de bases obtenu en coupant un acide nucléique présentant la séquence nucléotidique SEQ ID N°1 selon la revendication 1 par Sal I et Eco RI.

4. Séquence d'acide nucléique choisie parmi les séquences nucléotidiques SEQ ID N°1 et SEQ ID N°2 et leurs séquences complémentaires ainsi que les séquences qui en différent par mutation, insertion, délation ou substitution d'une ou plusieurs bases et qui hybrident spécifiquement avec une séquence d'acide nucléique génomique des souches appartenant au complexe Mycobactérium avium-intracellulaire et n'hybrident pas avec les acides nucléiques des mycobactéries n'appartenant pas audit complexe.

5. Amorces oligonucléotidiques pour l'amplification spécifique d'une séquence nucléique des souches appartenant au complexe Mycobacterium avium-intracellulare, caractérisées en ce qu'elles comprennent des paires d'oligonucléotides ayant de 18 à 30 nucléotides, de préférence de 18 à 22 nucléotides choisies parmi les séquences selon l'une des revendications 1 à 4.

6. Amorces oligonucléotidiques pour l'amplification spécifique d'une séquence nucléique des souches appartenant au complexe Mycobacterium avium-intracellulare selon la revendication 5, caractérisées en ce qu'elles comprennent des paires d'oligonucléotides d'au moins 18 nucléotides choisies parmi les séquences nucléotidiques représentées à la figure 1.

7. Amorces oligonucléotidiques pour l'amplification spécifique d'une séquence nucléique des souches appartenant au complexe Mycobacterium avium-intracellulare selon la revendication 5, caractérisées en ce qu'elles sont constituées par les paires oligonucléotidiques MA1 et MA2 d'une part et MA2 et MA3 d'autre part, de séquences :

MA1 : 5'GGC CCG ATG CGA CGT CGA GA3'
MA2 : 5'GCG CAA CTG CGC TTC GTC GA3'
MA3 : 5'GAA CGC CCG TTG CTG GCC ATT CAC GAG GAG3'
MA4 : 5'GCG CAA CAC GGT CGG ACA GGC CTT CCT CGA3'

8. Vecteur de clonage caractérisé en ce qu'il contient une séquence d'acide nucléique selon la revendication 4.

9. Plasmide p MA01 déposé à la CNCM le 28 août 1991 sous le n° I-1137.

10. plasmide p MA02 déposé à la CNCM le 28 août 1991 sous le n° I-1138.

11. Sondes nucléiques hybridant spécifiquement avec des séquences nucléiques des souches appartenant au complexe Mycobacterium avium-intracellulare, caractérisées en ce qu'elle comprennent au moins 20 nucléotides consécutifs choisis parmi les séquences nucléiques selon l'une des revendications 1 à 4.

12. Sondes nucléiques hybridant spécifiquement avec des séquences nucléiques des souches appartenant au complexe Mycobacterium avium-intracellulare, caractérisées en ce qu'elles comprennent les séquences nucléotidiques SEQ ID N°1, SEQ ID N°2, des fragments de ces séquences, des plasmides contenant ces séquences ou des fragments de ces séquences, des oligonucléotides complémentaires de ces séquences ou de ces fragments de séquences ou des produits d'amplification de ces séquences ou fragments de ces séquences.

13. Sondes nucléiques hybridant spécifiquement avec des séquences nucléiques des souches appartenant au complexe Mycobacterium avium-intracellulare, selon la revendication 12, caractérisées en ce qu'elles sont immobilisées sur un support et sont utilisées comme sondes de capture.

14. Méthode de détection de la présence des souches appartenant au complexe Mycobacterium avium intracellulare-dans un échantillon biologique, caractérisée par les étapes suivantes :
i) mise en contact de l'échantillon biologique avec un couple de fragments d'acide nucléique, dits amorces, selon l'une quelconque des revendications 5 à 7, l'acide nucléique des souches appartenant au complexe Mycobacterium avium-intracellulare contenu dans l'échantillon ayant été, le cas échéant, préalablement rendu accessible à l'hybridation et dans des conditions permettant une hybridation des amorces à l'acide nucléique des souches appartenant au complexe Mycobacterium avium-intracellulare ;
ii) amplification d'un acide nucléique des souches appartenant au complexe Mycobacterium avium intracellulare-;
iii) mise en évidence de l'amplification de fragments d'acide nucléique des souches appartenant au complexe Mycobacterium avium-intracellulare correspondant au fragment encadré par les amorces ;
iv) vérification éventuelle de la séquence du fragment amplifié, par exemple par hybridation de sonde spécifique, par séquençage ou par analyse de site de restriction.

15. Nécessaire pour la détection de la présence de souches appartenant au complexe Mycobacterium avium-intracellulare dans un échantillon biologique, caractérisé en ce qu'il comporte les éléments suivants :
- un couple de fragments d'acide nucléique selon l'une quelconque des revendications 5 à 7 ;
- les réactifs nécessaires pour effectuer une amplification d'un acide nucléique des souches appartenant au complexe Mycobacterium avium-intracellulare;
- éventuellement un composant permettant de vérifier la séquence du fragment amplifié, plus particulièrement une sonde nucléique selon l'une quelconque des revendications 11 à 13.

## Claims

1. A nucleic acid sequence which hybridizes specifically with a genomic nucleic acid sequence of strains of the Mycobacterium avium-intracellulare complex selected from the nucleotidic sequence SEQ ID No 1, the nucleotidic sequence SEQ ID No 2, complementary sequences thereto, and sequences which differ therefrom by the mutation, insertion, deletion or substitution of one or a plurality of bases, and which does not hybridize with the nucleic acids of mycobacteria which do not belong to the said complex.

2. A nucleic acid sequence which hybridizes specifically with a genomic nucleic acid sequence of the strains of the Mycobacterium avium-intracellulare complex having the serotypes 2, 3, 7, 12, 13, 14, 15, 16, 17, 18, 19, 20, 23, 24 and 25, and which does not hybridize with the nucleic acids of mycobacteria which does not belong to the said complex, **characterised in that** it comprises the nucleotidic sequence SEQ ID No. 2, according to Claim 1, a sequence complementary thereto, and sequences which differ therefrom by the mutation, insertion, deletion or substitution of one or a plurality of bases.

3. A nucleic acid sequence which hybridizes specifically with a genomic nucleic acid sequence of the strains of the M. avium complex having the serotype 1, 2, 3, 4, 5, 6, 8, 9, 10, 11 or 21 **characterised in that** it comprises a nucleic acid fragment of 700 base pairs which is obtained by cutting a nucleic acid having the nucleotidic sequence SEQ ID No. 1 according to Claim 1 by Sal I and Eco RI.

4. A nucleic acid sequence selected from the nucleotidic sequences SEQ ID No 1 and SEQ ID No 2 and the complementary sequences thereof and the sequences which differ therefrom by the mutation, insertion, deletion or substitution of one or a plurality of bases and which hybridize specifically with a genomic nucleic acid sequence of the strains of the Mycobacterium avium-intracellulare complex and which do not hybridize with the nucleic acids of mycobacteria which do not belong to the said complex.

5. Oligonucleotidic primers for specific amplification of a nucleic sequence of strains of the Mycobacterium avium-intracellulare complex **characterised in that** they comprise pairs of oligonucleotides having 18 to 30 nucleotides, preferably 18 to 22 nucleotides selected from the sequences according to any one of Claims 1 to 4.

6. Oligonucleotidic primers for specific amplification of a nucleic sequence of strains of the Mycobacterium avium-intracellulare complex according to Claim 5 **characterised in that** they comprise pairs of oligonucleotides with at least 18 nucleotides selected from the nucleotidic sequences shown in Figure 1.

7. Oligonucleotidic primers for specific amplification of a nucleic sequence of strains of the Mycobacterium avium-intracellulare complex according to Claim 5 **characterised in that** they comprise pairs of MA1 and MA2 oligonucleotides on one side and MA2 [sic] and MA3 [sic] oligonucleotides on the other, with sequences:

MA1 : 5'GGC CCG ATG CGA CGT CGA GA3'
MA2 : 5'GCG CAA CTG CGC TTC GTC GA3'
MA3 : 5'GAA CGC CCG TTG CTG GCC ATT CAC GAG GAG3'
MA4 : 5'GCG CAA CAC GGT CGG ACA GGC CTT CCT CGA3'

8. A cloning vector **characterised in that** it contains a nucleic acid sequence according to Claim 4.

9. Plasmid p MA01 deposited at the *CNCM* [National Collection of Micro-organism Cultures ] on 28 August 1991 under no I-1137.

10. Plasmid p MA02 deposited at the *CNCM* [National Collection of Micro-organism Cultures] on 28 August 1991 under no I-1138.

11. Nucleic probes hybridizing specifically with nucleic acid sequences of strains of the Mycobacterium avium-intracellulare complex **characterised in that** they comprise at least 20 consecutive nucleotides selected from the nucleic sequences according to any one of Claims 1 to 4.

12. Nucleic probes hybridizing specifically with nucleic acid sequences of strains of the Mycobacterium avium-intracellulare complex **characterised in that** they comprise the nucleotidic sequences SEQ ID no. 1, SEQ ID no. 2, fragments of these sequences, plasmids containing these sequences or fragments of these sequences, oligonucleotides complementary to these sequences or these fragments of sequences or amplification products of these sequences or fragments of these sequences.

13. Nucleic probes hybridizing specifically with nucleic acid sequences of strains of the Mycobacterium avium-intracellular complex according to Claim 12 **characterised in that** they are immobilised on a support and are used as capture probes.

14. A method for detecting the presence of strains of the Mycobacterium avium-intracellulare complex in a biological sample, **characterised by** the following stages
i) contacting of the biological sample with a pair of nucleic fragments, known as primers, according to any one of Claims 5 to 7, the nucleic acid of the strains of the Mycobacterium avium-intracellulare complex contained in the sample having first of all been, if necessary, made accessible to hybridization, and in conditions allowing hybridization of the primers to the nucleic acid of the strains of the Mycobacterium avium-intracellulare complex;
ii) amplifying of a nucleic acid of the strains of the Mycobacterium avium-intracellulare complex,
iii) demonstrating of the amplification of fragments of nucleic acid of the strains of the Mycobacterium avium-intracellulare complex corresponding to the fragment flanked by the primers,
iv) possible checking of the sequence of the amplified fragment, for example by specific probe hybridization, by sequencing or by analysing of the restriction site.

15. A kit for detecting the presence of strains of the Mycobacterium avium-intracellulare complex in a biological sample, **characterised in that** it comprises the following elements:
- a pair of nucleic acid fragments according to any one of claims 5 to 7;
- reagents required to amplify a nucleic acid of strains of the Mycobacterium avium-intracellulare complex, - possibly a component enabling checking of the amplified fragment sequence, specifically a nucleic probe according to any one of the claims 11 to 13.

## Patentansprüche

1. Nucleinsäuresequenz, welche spezifisch mit einer genomischen Nucleinsäuresequenz von Stämmen hybridisiert, die dem Komplex Mycobacterium avium-intracellulare angehören, ausgewählt aus der Nucleotidsequenz SEQ ID No. 1, der Nucleotidsequenz SEQ ID No. 2, komplementären Sequenzen davon, sowie Sequenzen, die sich davon durch Mutation, Einfügung, Entfernung oder Substitution einer oder mehrerer Basen unterscheiden, und die nicht mit Nucleinsäuren von Mycobacterien hybridisiert, die dem genannten Komplex nicht angehören.

2. Nucleinsäuresequenz, welche spezifisch mit der genomischen Nucleinsäure von Stämmen hybridisiert, die dem Komplex M. avium-intracellulare angehören, der die Serotypen 2, 3, 7, 12, 13, 14, 15, 16, 17, 18, 19, 20, 23, 24 und 25 trägt, hybridisiert und die nicht mit Nucleinsäuren von Mycobacterien hybridisiert, die dem genannten Komplex nicht angehören, dadurch gekennzeichnet, daß sie aus der Nucleotidsequenz SEQ ID No. 2 nach Anspruch 1, einer dazu komplementären Sequenz sowie Sequenzen, die sich davon durch Mutation, Einfügung, Entfernung oder Substitution einer oder mehrerer Basen unterscheiden, gebildet ist.

3. Nucleinsäuresequenz, welche spezifisch mit einer genomischen Nucleinsäuresequenz von Stämmen hybridisiert, die dem Komplex M. avium, der die Serotypen 1,2, 3, 4, 5, 6, 8, 9, 10, 11 oder 21 trägt, angehören, dadurch gekennzeichnet, daß sie aus einem Nucleinsäurefragment von 700 Basenpaaren gebildet ist, welches durch Spalten einer Nucleinsäure, welche die Nucleotidsequenz SEQ ID No. 1 nach Anspruch 1 aufweist, mit Sal I und Eco RI erhalten worden ist.

4. Nucleinsäuresequenz ausgewählt aus den Nucleotidsequenzen SEQ ID No. 1 und SEQ No. 2 und komplementären Sequenzen davon sowie Sequenzen, die sich davon durch Mutation, Einfügung, Entfernung oder Substitution einer oder mehrerer Basen unterscheiden, und die spezifisch mit einer genomischen Nucleinsäuresequenz von Stämmen hybridisiert, die dem Komplex Mycobacterium avium-intracellulare angehören und die nicht mit Nucleinsäuren von Mycobacterien hybridisiert, die dem genannten Komplex nicht angehören.

5. Oligonucleotid-Primer zur spezifischen Vermehrung einer Nucleinsequenz von Stämmen, die dem Komplex Mycobacterium avium-intracellulare angehören, dadurch gekennzeichnet, daß sie Oligonucleotidpaare mit 18 bis 30 Nucleotiden, vorzugsweise 18 bis 22 Nucleotiden ausgewählt aus den Sequenzen nach einem der Ansprüche 1 bis 4 umfassen.

6. Oligonunucleotid-Primer zur spezifischen Vermehrung einer Nucleinsequenz von Stämmen, die dem Komplex Mycobacterium avium-intracellulare angehören, nach Anspruch 5, dadurch gekennzeichnet, daß sie Oligonucleotidpaare mit mindestens 18 Nucleotiden ausgewählt aus den in der Figur 1 dargestellten Nucleotidsequenzen umfassen.

7. Oligonucleotid-Primer zur spezifischen Vermehrung einer Nucleinsäuresequenz von Stämmen, die dem Komplex Mycobacterium avium-intracellular angehören, nach Anspruch 5, dadurch gekennzeichnet, daß sie aus den Oligonucleotidpaaren MA1 und MA2 einerseits und MA2 und MA3 andereseits, der Sequenzen: 
MA1: 5'GGC CCG ATG CGA CGT CGA GA3'
MA2: 5'GCG CAA CTG CGC TTC GTC GA3'
MA3: 5'GAA CGC CCG TTG CTG GCC ATT CAC GAG GAG3'
MA4: 5'GCG CAA CAC GGT CGG ACA GGC CTT CCT CGA3'
 gebildet sind.

8. Klonierungsvektor, dadurch gekennzeichnet, daß er eine Nucleinsäuresequenz nach Anspruch 4 enthält.

9. Plasmid p MA01, das am 28. August 1991 unter der Nummer I-1137 beim CNCM hinterlegt worden ist.

10. Plasmid p MA02, das am 28. August 1991 unter der Nummer I-1138 beim CNCM hinterlegt worden ist.

11. Nucleinsonden, welche spezifisch mit Nucleinsequenzen von Stämmen hybridisieren, die dem Komplex Mycobacterium avium-intracellulare angehören, dadurch gekennzeichnet, daß sie mindestens 20 aufeinanderfolgende Nucleotide umfassen, ausgewählt aus den Nucleinsequenzen nach einem der Ansprüche 1 bis 4.

12. Nucleinsonden, die spezifisch mit Nucleinsequenzen von Stämmen hybridisieren, die dem Komplex Mycobacterium avium-intracellulare angehören, dadurch gekennzeichnet, daß sie die Nucleotidsequenzen SEQ ID No. 1, SEQ ID No. 2, Fragmente dieser Sequenzen, Fragmente dieser Sequenzen enthaltende Plasmide, komplemetäre Oligonucleotide von diesen Sequenzen oder von diesen Fragmenten von Sequenzen oder Vermehrungsprodukte dieser Sequenzen oder von Fragmenten dieser Sequenzen umfassen.

13. Nucleinsonden, die spezifisch mit Nucleinsequenzen von Stämmen hybridisieren, die den Komplex Mycobacterium avium-intracellulare angehören, nach Anspruch 12, dadurch gekennzeichnet, daß sie auf einem Träger immobilisiert sind und als Einfangsonden verwendet werden.

14. Verfahren zum Nachweis der Anwesenheit von Stämmen, die dem Komplex Mycobacterium avium-intracellulare angehören, in einer biologischen Probe, gekennzeichnet durch die folgenden Stufen:
i) Inkontaktbringen der biologischen Probe mit einem als Primer bezeichneten Paar von Nucleinsäurefragmenten nach einem der Ansprüche 5 bis 7, wobei die in der Probe enthaltene Nucleinsäure von Stämmen, die dem Komplex Mycobacterium avium-intracellulare angehören, gegebenenfalls zuvor der Hybridisierung zugänglich gemacht worden ist, und bei Bedingungen, die eine Hybridisierung der Primer mit der Nucleinsäure von Stämmen, die dem Komplex Mycobacterium avium-intracellulare angehören, ermöglichen;
ii) Vermehrung einer Nucleinsäure von Stämmen, die dem Komplex Mycobacterium avium-intracellulare angehören;
iii) Nachweis der Vermehrung von Nucleinsäurefragmenten von Stämmen, die dem Komplex Mycobacterium avium-intracellulare angehören, welche dem von den Primern umfaßten Fragment entsprechen;
iv) eventuelle Verifizierung der Sequenz des vermehrten Fragments beispielsweise durch Hybridisierung der spezifischen Sonde, durch Sequenzierung oder durch restruktionelle Analyse.

15. Reagenz für den Nachweis der Anwesenheit von Stämmen, die dem Komplex Mycobacterium avium-intracellulare angehören, in einer biologischen Probe, dadurch gekennzeichnet, daß es die folgenden Elemente umfaßt:
- ein Paar von Nucleinsäurefragmenten nach einem der Ansprüche 1 bis 7;
- die erforderlichen Reagenzien zur Durchführung einer Vermehrung einer Nucleinsäure von Stämmen, die dem Komplex Mycobacterium avium-intracellulare angehören;
- gegebenenfalls eine Einrichtung, welche es ermöglicht, die Sequenz des vermehrten Fragments zu verifizieren, insbesondere eine Nucleinsonde nach einem der Ansprüche 11 bis 13.
